# EUROPEAN PATENT APPLICATION

(11) **EP 4 722 384 A1**
(43) Date of publication of application: **08.04.2026**
(21) Application number: 24204193.7
(22) Date of filing: 02.10.2024
(51) Int. Cl.: C12Q 1/6851

(54) **DPCR WITH EXTENDED DYNAMIC RANGE**

(71) Applicant: QIAGEN GmbH, 40724 Hilden (DE)
(72) Inventor: Matthaei, Kevin, 40724 Hilden (DE); Bielemeier, Celia, 40724 Hilden (DE); Weide, Deborah, 40724 Hilden (DE); Endres, Christopher, 40724 Hilden (DE)
(74) Representative: Hoffmann Eitle

(57) **Abstract**

The present invention provides methods for quantification of target nucleic acid molecules. In particular, improved digital polymerase chain reaction (dPCR) methods are provided with a broadened dynamic range. Furthermore, the present invention provides systems and computer-readable storage media for said dPCR methods.

## Description

### FIELD OF THE INVENTION

The present invention provides improved methods for quantification of target nucleic acid molecules such as digital polymerase chain reaction (dPCR). Furthermore, the present invention provides a system and a computer-readable storage medium for performing said methods.

### BACKGROUND OF THE INVENTION

Quantitative PCR (qPCR), or real time PCR, is used to detect, characterize and quantify nucleic acids for numerous applications. As in standard PCR, DNA is amplified by three repeating steps: denaturation, annealing, and elongation. In qPCR, probes labelled with fluorescent dye enable the collection of fluorescence data allowing to monitor the PCR progress. In order to be able to quantify the concentration of the tested template qPCR requires the use of standards.

In contrast, in digital PCR (dPCR) each sample containing a small number of target nucleic acids is divided into lots of subsamples, also called partitions. Consequently, each partition either contains the target nucleic acid or not. Subsequently, an end-point PCR according to standard protocols is executed and a signal for each partition is measured. For instance, for a partition containing the target nucleic acid a positive detection signal may be measured due to an amplification reaction while for a partition that does not contain the target nucleic acid no detection signal is generated since no amplification reaction occurred. Thus, dPCR uses the standard procedure of end-time PCR, but splits the PCR in many single partitions in which the target is randomly distributed across all available partitions. As the target is distributed randomly, the amount of target nucleic acid in the sample can be calculated using Poisson statistics. Such dPCR systems combine high-throughput screening and multiplexing capabilities with high sensitivity, excellent precision and very good reproducibility. Several dPCR systems are commercially available, e.g. the QIAcuity systems (QIAGEN, Hilden), which are designed for applications as rare mutation detection, copy number variation, gene expression analysis, NGS (next generation sequencing) library quantification, pathogen detection, viral load detection, genotyping, miRNA research, IncRNA analysis, and genetically modified organisms (GMO) detection.

The process of partitioning, thermocycling, imaging (i.e., data collection), and analysis is commonly implemented in a biological analysis system, such as the Q!Acuity instrument (QIAGEN, Hilden). These systems at least include a thermal cycler, an optical system, one or more processors, a memory, a storage device, and a display. In dPCR, the signals are usually fluorescence signals emitted from fluorophores and detected in different channels according to the wavelength of the emitted signals. Typically, one or more fluorophores are used. Based on all signals detected in one channel, a threshold can be calculated to distinguish between positive and negative partitions in that channel, corresponding to the partitions where an amplification reaction occurred or not. As a result, these systems provide the concentration in copies per microliter of the target nucleic acids as well as quality control such as positive samples or non-template controls (NTC).

dPCR has the advantage that, unlike qPCR, no standard is required for concentration estimation. However, qPCR allows a wider analytical range of concentration up to the power of 10^8, while in dPCR the dynamic range which can be achieved depends e.g. on the overall number of partitions. Due to the dependence on the ratio of negative partitions, the dPCR concentration range of analysis can be quickly limited in the upper range of concentration, namely when all partitions are positive. In this case, concentration estimation cannot take place. For example, if a reporter gene's signal is close to saturation while the signal of the gene of interest is close to the limit of detection, estimation of template concentration may not be possible using conventional dPCR. In particular, in such a case, dilution of the sample would not be possible because the signal for the gene of interest would be lost.

Therefore, there is a need in the art to provide dPCR methods with an extended dynamic range which are in particularly suitable to quantify also high concentration samples.

### SUMMARY OF THE INVENTION

The present invention overcomes core drawbacks of the prior art. In particular, the present invention provides improved methods for quantifying target nucleic acid molecules, especially via digital PCR (dPCR), with an extended dynamic range which allow for analysis of highly concentrated samples. The methods according to the present invention enable determination of target concentration even in saturated samples by use of an intermediate detection step at a PCR cycle at which the endpoint signal has not yet been reached. The detection of the signal at said intermediate timepoint in addition to the detection of the signal at the end point of the dPCR allows assessment of the concentration of high copy targets that result in only few or even no negative partitions in standard dPCR.

The methods according to the invention are in particular suitable for the detection of high copy target nucleic acids in biological samples in which a further target nucleic acid is only present in minimal quantities. Thus, the method provided herein combines the greater accuracy of dPCR compared to traditional real-time PCR with an extended dynamic range for analysis of saturated biological samples. The present invention therefore makes an important contribution to the art.

According to a first aspect, the present invention provides a method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint.

In a second aspect, the present invention provides a method of quantitating first target nucleic acid molecules and second target nucleic acid molecules in a sample comprising:
a) providing a sample comprising first target nucleic acid molecules which contain a first target nucleic acid sequence, and second target nucleic acid molecules which contain a second target nucleic acid sequence which is different from the first target nucleic acid sequence; and
b) separating said sample into a plurality of partitions; and
c) subjecting said plurality of partitions to multiple cycles of polymerase chain reaction (PCR cycles) for amplification of said first target nucleic acid sequence to produce first target amplicons in one or more of said partitions, and for amplification of said second target nucleic acid sequence to produce second target amplicons in one or more of said partitions; and
d) determining the intensity of a first detectable signal in said plurality of partitions
   (i) at a first determination timepoint during the exponential phase of the amplification, and
   (ii) at a second determination timepoint during a stationary phase of the amplification,

   wherein the first detectable signal correlates to the amount of said first target amplicons; and
   determining the number of partitions which are positive for said second target amplicons, wherein a positive partition has an intensity of a second detectable signal at a determination timepoint during the plateau phase of the amplification which is above a threshold value, wherein the second detectable signal correlates to the amount of said second target amplicons; and
e) calculating an initial number of first target nucleic acid molecules which contain said first target nucleic acid sequence that are present in said sample before said sample has been separated into said plurality of partitions, wherein said calculation is based on the relation between the intensity of the first detectable signal determined at the first determination timepoint and the intensity of the first detectable signal determined at the second determination timepoint; and
   calculating an initial number of second target nucleic acid molecules which contain said second target nucleic acid sequence that are present in said sample before said sample has been separated into said plurality of partitions based on the number or ratio of positive partitions.

The method according to the second aspect is in particular suitable for samples wherein the amount of the first target nucleic acid is significantly higher than the amount of the second target nucleic acid. Especially, while the amount of the second target nucleic acid is in the range suitable for standard dPCR, the amount of the first target nucleic acid may be above the dynamic range of standard dPCR.

According to a third aspect, the present invention provides a system for performing the method according to the first or second aspect, comprising:
(a) at least one processor for performing the method according to the first or second aspect; and
(b) a memory encoded with instructions to perform the method according to the first or second aspect.

According to a fourth aspect, the present invention provides a computer-readable storage medium encoded with instructions, executable by a processor, for performing the method according to the first or second aspect.

The system according to the third aspect and/or the computer-readable storage medium according to the fourth aspect may be used in the method according to the first or second aspect.

Other objects, features, advantages and aspects of the present invention will become apparent to those skilled in the art from the following description and appended claims. It should be understood, however, that the following description, appended claims, and specific examples, which indicate preferred embodiments of the application, are given by way of illustration only. Various changes and modifications within the spirit and scope of the disclosed invention will become readily apparent to those skilled in the art from reading the following.

### BRIEF DESCRIPTION OF THE FIGURES

**Figure 1** shows the mean relative fluorescence units (RFU) of all partitions measured every fifth cycle for the different concentration of the target nucleic acid molecules (BRAF V600 assay) in the sample.
**Figure 2** shows a logarithmic concentration curve depending on the Ct value (RFU of 40) as number of cycles plotted against concentration.
**Figure 3** shows a logarithmic concentration curve depending on the ratio of the RFU determined at cycle 25 and cycle 50 as RFU ratio (25/50) plotted against concentration in logarithmic scale.
**Figure 4** shows standard curves of the RFU ratio (25/50) plotted against the concentration of the standard template in the sample for three different instruments.
**Figure 5** shows standard curves of the RFU ratio (25/50) plotted against the concentration of the high copy target nucleic acid molecules (BRAF V600 assay) in the sample which additionally comprises a low copy target nucleic acid.
**Figure 6** shows the fluorescence signals detected by Q!Acuity software Suite in the end point imaging step (after 50 cycles) for the low copy target (EGFR, left) and the high copy target (BRAF V600 assay, right).
**Figure 7** shows standard curves of the RFU ratio (25/50) plotted against the concentration of the target nucleic acid molecules (BRAF V600 assay) present in the sample in high or low copy number.

### DETAILED DESCRIPTION OF THE INVENTION

The different aspects and embodiments of the invention disclosed herein make important contributions to the art as is also explained in the following.

### The method according to the first aspect

According to a first aspect, the present invention provides a method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint.

By determining the intensity of the detectable signal in said plurality of partitions during the exponential phase of the amplification, the amount of target nucleic acid molecules in the sample can be calculated even if the number of negative partitions in the dPCR is too low to use Poisson statistics. The ratio of the intensity of the detectable signal at the first determination timepoint divided by the intensity of the detectable signal at the second determination timepoint corresponds to a certain amount of target nucleic acid molecules in the sample. Over a large concentration range of the target nucleic acid molecule in the sample, there is a linear relationship between said ratio and the concentration, especially for concentrations which are too high for Poisson statistics. This correlation may be determined by one or more test runs or may be known from previous experience. Thus, using only a single additional measurement during the exponential phase, the dynamic range of a dPCR is significantly widened to higher concentration ranges by the method according to the present invention.

### Step a)

In certain embodiments, the sample is a biological sample. The sample may contain living cells and/or dead cells and/or lysed cells. In other embodiments, the sample does not contain any cells. The sample may be obtained from a biological organism, from the environment, or from an artificial process or product. The sample may in particular be selected from the group consisting of a human sample, an animal sample, a plant sample, a microbiological sample, a wastewater sample and a cell culture sample.

The sample may be a bodily sample, especially a liquid body sample, such as blood, serum, plasma, or saliva, or a solid body sample, such as a biopsy from any tissue. Furthermore, the sample may be a swab including nasal swabs, oral swabs, nasopharyngeal swabs and oropharyngeal swabs. The sample may be obtained from a human subject suspected to be infected with a pathogen or to have a genetic disorder. Furthermore, the sample may comprise human material, such as human cells or cell components, e.g. human nucleic acids.

The sample comprises target nucleic acid molecules which contain a target nucleic acid sequence. The nucleic acid molecules may comprise or substantially consist of DNA, including genomic DNA, human DNA, pathogenic DNA, viral DNA and environmental DNA (eDNA). Alternatively, the nucleic acids may comprise or substantially consist of RNA, including mRNA, miRNA, siRNA, IncRNA, human RNA, pathogenic RNA and viral RNA. In certain embodiments, the target nucleic acid molecules are DNA. In alternative embodiments, the target nucleic acid molecules are RNA. The target nucleic acid molecules may encode a protein of interest. In certain embodiments, the target nucleic acid molecules originate from a pathogen.

The target nucleic acid molecules present in the sample may be identical or different. A nucleic acid molecule is a target nucleic acid molecule if it contains the target nucleic acid sequence. The sample may further contain other nucleic acid molecules which do not contain the target nucleic acid sequence.

In certain embodiments, the target nucleic acid sequence is indicative for the presence of a certain pathogen, organism, condition of an organism, microorganism, vector, or plasmid. The target nucleic acid sequence may for example be indicative for a certain disease of an organism such as a human being. In these embodiments, the target nucleic acid sequence may be indicative for the presence of a pathological microorganism, including bacteria, fungi and viruses, or of genetically defect cells of the organism, including cancer cells and cells with a congenital gene defect.

The target nucleic acid sequence may have a length of at least 10 nucleotides. In certain embodiments, the target nucleic acid sequence has a length of at least 15 nucleotides, especially at least 18 nucleotides. The target nucleic acid sequence may for example have a length of about 10 to about 2000 nucleotides. In specific embodiments, the target nucleic acid sequence has a length of 18 to 25 nucleotides.

### Step b)

In certain embodiments, the partitions are spatially isolated partitions. The partitions may be liquid partitions or solid partitions. Exemplary liquid partitions are droplet, for example aqueous droplets in a lipid phase, or liquid volumes in separate chambers. Exemplary solid partitions are beads or surface areas on a chip. Solid partitions may have binding moieties which immobilize nucleic acids on their surface. The partitions may be present in through-holes, wells, indentations, spots, cavities, sample retainment regions, or reaction chambers. In certain embodiments, the sample is separated in step b) into at least 100 partitions, at least 1000 partitions, at least 10⁴, 10⁵, 10⁶ or 10⁷ partitions.

Separation of the samples into the plurality of partitions is preferably performed in an automized manner. Each of the plurality of partitions may have the same size, or different partitions may have different sizes. In one embodiment, all partitions have the same size. In another embodiment, different subsets of partitions are formed wherein the partitions within a subset have the same size, but partitions of different subsets have different sizes.

The target nucleic acid molecules present in a sample are in particular evenly and/or randomly distributed over the plurality of partitions in step b). In certain embodiments, each of the partitions in step b) contains on average more than one target nucleic acid molecules. Especially, each of said partitions in step b) contains on average five or more target nucleic acid molecules. For example, each of said partitions in step b) contains on average ten or more target nucleic acid molecules. In certain embodiments, the portion of said plurality of partitions that contains zero target nucleic acid molecules is insufficient in number to allow application of Poisson statistics. For example, the portion of said plurality of partitions that contains zero target nucleic acid molecules is 0.2% or less, 0,1% or less, preferably 0.02% or less or 0.01% or less.

### Step c)

In step c), the plurality of partitions are subjected to multiple cycles of polymerase chain reaction (PCR cycles) for amplification of said target nucleic acid sequence. The partitions in particular comprise amplification reagents sufficient to perform a polymerase chain reaction at the beginning of step c). The amplification reagents may be added to the partitions after separation of the sample in step b), or they may be added to the sample before its separation in step b), or the sample provided in step a) may already contain the amplification reagents. In certain embodiments, the method comprises the further step of adding amplification reagents between steps a) and b) or between steps b) and c). The amplification reagents may in particular comprise primer nucleic acid molecules specific for the target nucleic acid sequence, nucleotides and a polymerase.

In certain embodiments, each of the PCR cycles comprises the steps of denaturing nucleic acid duplexes, hybridizing primer nucleic acid molecules with the target nucleic acid sequence, and elongating the hybridized primer nucleic acid molecules by a polymerase. Performing the PCR cycles results in amplification of the target nucleic acid sequence to produce target amplicons in one or more of said partitions. In certain embodiments, in step c) the plurality of partitions are subjected to at least 20 PCR cycles, preferably at least 25, at least 30, at least 35, at least 40, at least 45, at least 50, at least 55, or at least 60 PCR cycles.

In certain embodiments, in each partition a PCR is performed leading to amplification of the target nucleic acid molecule(s) if present in partition. If an amplification reaction occurs, a signal, such as a fluorescence signal, can be detected in the respective partition indicating the presence of the at least one target nucleic acid molecule in the respective partition. In case no amplification reaction occurs, no signal is detected indicating that no target nucleic acid molecule is present in the respective partition.

### Step d)

In step d), the intensity of a detectable signal is determined in said plurality of partitions. The detectable signal may be any signal which can be detected and which correlates to the amount of the target amplicons produced in the partitions in step c). In certain embodiments, the detectable signal is an optical signal, in particular a fluorescence signal.

The partitions in particular comprise detection reagents sufficient to generate a detectable signal in the presence of produce target amplicons at the beginning of step d). The detection reagents may be added to the partitions after separation of the sample in step b), or they may be added to the sample before its separation in step b), or the sample provided in step a) may already contain the detection reagents. In certain embodiments, the method comprises the further step of adding detection reagents between steps a) and b) or between steps b) and c). In certain embodiments, the detection reagents are added to the partitions together with the amplification reagents. In these embodiments, the method may comprise the further step of adding amplification reagents and detection reagents between steps a) and b) or between steps b) and c). In certain embodiments, each of said partitions when being subjected to the initial PCR cycle comprises detection reagents which produce the detectable signal.

The detection reagents may in particular comprise a label, especially an optical label, which emits the detectable signal in the presence of the target amplicons. The label may in particular be a fluorescent label and the detectable signal a fluorescence signal. The detectable signal correlates to the amount of the target amplicons.

In step d), the intensity of a detectable signal is determined in said plurality of partitions at a first determination timepoint during the exponential phase of the amplification.

In certain embodiments, in the exponential phase the amount of target amplicons is about 10% to about 90% of the amount of target amplicons at the end of the last PCR cycle. In further embodiments, in the exponential phase the amount of target amplicons is about 20% to about 80% of the amount of target amplicons at the end of the last PCR cycle.

In certain embodiments, in the exponential phase the intensity of the detectable signal is between about 10% and about 90%, with the detectable signal at the first cycle being 0% and the detectable signal at last cycle being 100%. In further embodiments, in the exponential phase the intensity of the detectable signal is between about 20% and about 80%, with the detectable signal at the first cycle being 0% and the detectable signal at last cycle being 100%.

In certain embodiments, the first determination timepoint is during or directly after any one of PCR cycles 10 to 35. In preferred embodiments, the first determination timepoint is during or directly after any one of PCR cycles 20 to 30. Exemplary cycles, during or directly after which the first determination timepoint is, include for example PCR cycles 22, 23, 24, 25, 26, 27 and 28, especially PCR cycle 24, 25 and 26.

In step d), the intensity of a detectable signal is further determined in said plurality of partitions at a second determination timepoint during a stationary phase of the amplification. The second determination timepoint may be before or after the first determination timepoint. Between the first determination timepoint and the second determination timepoint, the plurality of partitions are subjected to at least one PCR cycle of step c). In certain embodiments, the plurality of partitions are subjected to at least two, at least three, at least four or at least five PCR cycles of step c) between the first determination timepoint and the second determination timepoint.

The stationary phase of the amplification, in which the second determination timepoint lies, includes the plateau phase of the amplification and the baseline phase of the amplification. The baseline phase is at the beginning of the amplification before the exponential phase and the plateau phase is at the end of the amplification after the exponential phase. The baseline phase in particular comprises consecutive cycles starting with cycle 1 of the PCR and ending once the detectable signal significantly rises. A significant rise of the detectable signal in this respect for example is a rise by at least 5%, wherein the detectable signal at the last cycle being 100%. The plateau phase in particular comprises consecutive cycles starting once the detectable signal ceases to significantly rise after the exponential phase and ending with the last cycle of the PCR. A significant rise of the detectable signal in this respect for example is a rise by at least 5%, wherein the detectable signal at the last cycle being 100%.

In certain embodiments, the second determination timepoint is during the plateau phase of the amplification.

In certain embodiments, in the plateau phase the amount of target amplicons is more than about 80% of the amount of target amplicons at the end of the last PCR cycle. In further embodiments, in the plateau phase the amount of target amplicons more than about 90% of the amount of target amplicons at the end of the last PCR cycle. In certain embodiments, in the plateau phase the intensity of the detectable signal is more than about 80%, with the detectable signal at the first cycle being 0% and the detectable signal at the last cycle being 100%. In further embodiments, in the plateau phase the intensity of the detectable signal is more than about 90%, with the detectable signal at the first cycle being 0% and the detectable signal at the last cycle being 100%.

In certain embodiments, the second determination timepoint is during or directly after any one of PCR cycles 30 to 60. In preferred embodiments, the second determination timepoint is during or directly after any one of PCR cycles 45 to 55. Exemplary cycles, during or directly after which the first determination timepoint is, include for example PCR cycles 45, 50 and 55.

In certain embodiments, the second determination timepoint is during the baseline phase of the amplification.

In certain embodiments, in the baseline phase the amount of target amplicons is less than about 20% of the amount of target amplicons at the end of the last PCR cycle. In further embodiments, in the baseline phase the amount of target amplicons less than about 10% of the amount of target amplicons at the end of the last PCR cycle. In certain embodiments, in the baseline phase the intensity of the detectable signal is less than about 20%, with the detectable signal at the first cycle being 0% and the detectable signal at the last cycle being 100%. In further embodiments, in the baseline phase the intensity of the detectable signal is less than about 10%, with the detectable signal at the first cycle being 0% and the detectable signal at the last cycle being 100%.

In certain embodiments, the second determination timepoint is during or directly after any one of PCR cycles 1 to 20. In preferred embodiments, the second determination timepoint is during or directly after any one of PCR cycles 1 to 15. Exemplary cycles, during or directly after which the first determination timepoint is, include for example PCR cycles 1, 2, 3, 4, 5 and 10.

In certain embodiments, the intensity of the detectable signal is only determined twice in the plurality of partitions. In these embodiments, the method only comprises determining the intensity of the detectable signal at the first determination timepoint and at the second determination timepoint, and excludes any further determination of the intensity of the detectable signal at another timepoint.

In certain embodiments, the intensity of the detectable signal is only determined thrice in the plurality of partitions. In these embodiments, the method only comprises determining the intensity of the detectable signal at the first determination timepoint during the exponential phase of the amplification, at the second determination timepoint during the plateau phase of the amplification and at a third determination timepoint during the baseline phase of the amplification, and excludes any further determination of the intensity of the detectable signal at another timepoint.

In certain embodiments, the first determination timepoint and the second determination timepoint are separated by at least 3 PCR cycles. In particular, the first determination timepoint and the second determination timepoint are separated by at least 5 PCR cycles, especially by at least 10 PCR cycles.

In certain embodiments, the intensity of the detectable signal is determined in the plurality of partitions at one or more further determination timepoints different from the first and second determination timepoint. The different determination timepoints are separated from each other by at least one PCR cycle, in particular by at least 2 PCR cycle. In certain embodiments, the intensity of the detectable signal is determined in the plurality of partitions periodically every third PCR cycle, every fourth PCR cycle, or every fifth PCR cycle. One or more of the further determination timepoints may be during the exponential phase of the amplification.

In certain embodiments, positions of the first determination timepoint and/or the second determination timepoint during the amplification are determined by one or more test runs. For example, in a test run preset position are used for the first determination timepoint and the second determination timepoint, such as during or directly after cycles 25 and 50. If the relative value obtained by dividing the intensity of the detectable signal determined at the first determination timepoint by the intensity of the detectable signal determined at the second determination timepoint is not within a desired range, the position of the first determination timepoint and/or the second determination timepoint may be changed. For example, if the relative value is too low, the first determination timepoint and/or the second determination timepoint may be moved to a later cycle; and if the relative value is too high, the first determination timepoint and/or the second determination timepoint may be moved to an earlier cycle.

In alternative embodiments, preset positions of the first determination timepoint and the second determination timepoint during the amplification are used. These preset positions may in particular be determined by one or more standard runs where the method is performed with standard samples having a known amount of target nucleic acid molecules. Preferably, at least 5 standard runs with standard samples having different known amount of target nucleic acid molecules are performed to determine the set positions of the first determination timepoint and the second determination timepoint during the amplification. The used standard samples in particular cover a range of different amounts of at least one order of magnitude.

The intensity of the detectable signal in the plurality of partitions may be determined in any suitable manner. For example, a common intensity of the detectable signal may be determined for all partitions of the plurality of partitions. In this embodiment, the cumulative detectable signal of all partitions may be determined by one measurement. Alternatively, individual intensities of the detectable signal may be measured for each partition of the plurality of partitions. In this embodiment, the individual intensities of the partitions may be added up to provide a total intensity, or the individual intensities of the partitions may be used to calculate a mean or median intensity.

In certain embodiments, the intensities of the detectable signal determined at the first and second determination timepoints and optionally at any further determination timepoints are corrected by a reference intensity. The reference intensity may for example be the intensity of the detectable signal determined at a timepoint during the baseline phase of the amplification.

### Step e)

In step e), an initial number of target nucleic acid molecules that are present in the initial sample are calculated. The initial sample is the sample provided in step a) before it has been separated into said plurality of partitions in step b). The calculation is based on the relation between the intensity of the detectable signal determined at the first determination timepoint and the intensity of the detectable signal determined at the second determination timepoint.

In certain embodiments, the calculation involves a relative value obtained by dividing the intensity of the detectable signal determined at the first determination timepoint by the intensity of the detectable signal determined at the second determination timepoint. Calculating the initial number of target nucleic acid molecules which contain said target nucleic acid sequence may in particular comprise comparing the relative value to a reference curve or to reference relative values.

The reference relative values and/or the reference curve may be based on at least two reference samples comprising a known number of reference nucleic acid molecules. A reference relative value is obtained for each reference sample, wherein reference amplicons are produced from the reference nucleic acid molecules by the same method steps b) and c) as for the target amplicons, and wherein reference relative values are determined by the same method steps d) and e) as the relative value for the target amplicons. The reference relative values and/or the reference curve may in particular be based on at least three, especially at least four or at least five, reference samples comprising a known number of reference nucleic acid molecules. The reference samples may comprise different numbers of reference nucleic acid molecules. The reference curve may in particular be a plot of the reference relative values of the reference samples against the known number of reference nucleic acid molecules in the reference samples.

In certain embodiments, the reference nucleic acid molecules contain the same target nucleic acid sequence as the target nucleic acid molecules. In these embodiments, the reference amplicons are preferably produced by amplification of said target nucleic acid sequence present on the reference nucleic acid molecules.

In certain embodiments, the calculation in step e) involves intensities of the detectable signal in said plurality of partitions which are determined at exactly two determination timepoints during the amplification, namely at the first determination timepoint and at the second determination timepoint. In particular, the calculation in step e) does not involve the intensity of the detectable signal in said plurality of partitions which is determined at another determination timepoint during the amplification which is different from the first determination timepoint and the second determination timepoint.

In certain embodiments, the calculation in step e) involves intensities of the detectable signal in said plurality of partitions which are determined at exactly three determination timepoints during the amplification, namely at the first determination timepoint, at the second determination timepoint and at a third determination timepoint during the baseline phase of the amplification. In particular, the calculation in step e) does not involve the intensity of the detectable signal in said plurality of partitions which is determined at another determination timepoint during the amplification which is different from the first determination timepoint, the second determination timepoint and at a third determination timepoint during the baseline phase of the amplification.

In certain embodiments, the method is simultaneously performed for two or more different target nucleic acid sequences. In particular, for each different target nucleic acid sequence respective target amplicons are produced in step c), and for each different target amplicon a different detectable signal is used in step d) which correlates to the amount the respective target amplicon.

Calculating the initial number of target nucleic acid molecules which contain the respective target nucleic acid sequence that are present in the initial sample may be done for each target nucleic acid sequence based on the relation between the intensity of the corresponding detectable signal determined at the first determination timepoint and the intensity of the corresponding detectable signal determined at the second determination timepoint. Different reference relative values and/or reference curves may be used for each different target nucleic acid sequence, or the same reference relative values and/or reference curve may be used for two or more, such as for all, different target nucleic acid sequences.

### The method according to the second aspect

In a second aspect, the present invention provides a method of quantitating first target nucleic acid molecules and second target nucleic acid molecules in a sample comprising:
a) providing a sample comprising first target nucleic acid molecules which contain a first target nucleic acid sequence, and second target nucleic acid molecules which contain a second target nucleic acid sequence which is different from the first target nucleic acid sequence; and
b) separating said sample into a plurality of partitions; and
c) subjecting said plurality of partitions to multiple cycles of polymerase chain reaction (PCR cycles) for amplification of said first target nucleic acid sequence to produce first target amplicons in one or more of said partitions, and for amplification of said second target nucleic acid sequence to produce second target amplicons in one or more of said partitions; and
d) determining the intensity of a first detectable signal in said plurality of partitions
   (i) at a first determination timepoint during the exponential phase of the amplification, and
   (ii) at a second determination timepoint during a stationary phase of the amplification,
      wherein the first detectable signal correlates to the amount of said first target amplicons; and
      determining the number of partitions which are positive for said second target amplicons, wherein a positive partition has an intensity of a second detectable signal at a determination timepoint during the plateau phase of the amplification which is above a threshold value, wherein the second detectable signal correlates to the amount of said second target amplicons; and
e) calculating an initial number of first target nucleic acid molecules which contain said first target nucleic acid sequence that are present in said sample before said sample has been separated into said plurality of partitions, wherein said calculation is based on the relation between the intensity of the first detectable signal determined at the first determination timepoint and the intensity of the first detectable signal determined at the second determination timepoint; and
   calculating an initial number of second target nucleic acid molecules which contain said second target nucleic acid sequence that are present in said sample before said sample has been separated into said plurality of partitions based on the number or ratio of positive partitions.

The features and embodiments of the method according to the first aspect likewise apply to the method according to the second aspect. The features and embodiments concerning the target nucleic acid molecules, the target nucleic acid sequence, the target amplicons and the detectable signal of the method according to the first aspect in particular apply to the first target nucleic acid molecules, the first target nucleic acid sequence, the first target amplicons and the first detectable signal of the method according to the second aspect.

In certain embodiments, the amount of first target nucleic acid molecules in the sample is higher than the amount of second target nucleic acid molecules. Especially, the amount of first target nucleic acid molecules is at least 2-fold higher, in particular at least 10-fold higher, at least 100-fold higher, at least 1000-fold higher, at least 10⁵ higher, at least 10⁶ higher, or at least 10⁷ higher. The initial number of second target nucleic acid molecules may thus be lower than the initial number of first target nucleic acid molecules.

In certain embodiments, each of the partitions in step b) contains on average less than five second target nucleic acid molecules, in particular on average about one or fewer second target nucleic acid molecules.

In certain embodiments, the second detectable signal is different from the first detectable signal.

In certain embodiments, in step d) the threshold value used for identifying positive partitions is indicative of one second target nucleic acid molecule being present in said partition in step b). In these embodiments, if a partition does not contain a second target nucleic acid molecule, then the intensity of the second detectable signal at said determination timepoint during the plateau phase of the amplification is below said threshold value. Such a partition may be determined as negative partition.

In certain embodiments, a partition has to fulfill one or more additional criteria in addition to the second detectable signal being above the threshold value at said determination timepoint during the plateau phase of the amplification in order to qualify as positive partition. Exemplary criteria in this respect may be the intensity of the second detectable signal being below a further threshold value at a determination timepoint during the baseline phase of the amplification, and/or the intensity of the second detectable signal being above a further threshold value at a determination timepoint during the exponential phase of the amplification.

In certain embodiments, a partition has to fulfill one or more additional criteria in addition to the second detectable signal being below the threshold value at said determination timepoint during the plateau phase of the amplification in order to qualify as negative partition. Exemplary criteria in this respect may be the intensity of the second detectable signal being below a further threshold value at a determination timepoint during the baseline phase of the amplification, and/or the intensity of the second detectable signal being below a further threshold value at a determination timepoint during the exponential phase of the amplification.

In certain embodiments, the intensity of the second detectable signal is determined at the second determination timepoint. In these embodiments, the second determination timepoint in particular is during the plateau phase of the amplification reaction.

In certain embodiments, the initial number of second target nucleic acid molecules which contain the second target nucleic acid sequence is calculated by application of Poisson statistics.

### The system according to the third aspect

According to a third aspect, the present invention provides a system for performing the method according to the first or second aspect, comprising:
(a) at least one processor for performing the method according to the first or second aspect; and
(b) a memory encoded with instructions to perform the method according to the first or second aspect.

In certain embodiments, the system according to the third aspect further comprises
(c) a display;
(d) a thermal cycler;
(e) an optical system;
(f) a storage device; and
(g) optionally an input device.

In certain embodiments, the components (a) to (b) or (a) to (g) are connected to communicate and process information. The system may include bus or other communication mechanism for communicating information, and processor coupled with bus for processing information.

According to one embodiment, the memory is a dynamic memory, preferably a random access memory (RAM). According to one embodiment, the memory is a static storage device, such as a read only memory (ROM).

According to one embodiment, the display comprises an input device. According to one embodiment, the display is a touchscreen. An input device may be an alphanumeric and other keys, which is coupled to bus for communicating information and command selections to processor, a cursor control, such as a mouse, a trackball or cursor direction keys for communicating information and command selections to processor and for controlling cursor movement on display.

The system provides data processing, such as dPCR data processing. Data processing according to the method of the first aspect is provided by the system in response to processor executing one or more sequences of one or more instructions to perform the method according to the first aspect contained in memory. Such instructions may be read into memory from another computer-readable medium, such as storage device.

### The storage medium according to the fourth aspect

According to a fourth aspect, the present invention provides a computer-readable storage medium encoded with instructions, executable by a processor, for performing the method according to the first or second aspect.

The term "computer-readable storage medium" as used herein generally refers to any media that is involved in providing one or more sequences or one or more instructions for performing the method according to the first or second aspect to processor for execution enabling the system to perform features or functions of embodiments of the present invention.

According to one embodiment, the computer-readable storage medium is selected from the group consisting of non-volatile media, volatile media, and transmission media. The computer-readable storage medium can be read by a device which is connected to the processor by direct hardware or the computer-readable storage medium may be stored remote to the device, especially in a cloud to which the processor can have access.

### Use according to the fifth aspect

According to a fifth aspect, a use of the system according to the third aspect to perform the method according to the first or second aspect is provided. Furthermore, a use of the computer-readable storage medium according to the fourth aspect to perform the method according to the first or second aspect is provided.

The expression "comprise", as used herein, besides its literal meaning also includes and specifically refers to the expressions "consist essentially of" and "consist of". Thus, the expression "comprise" refers to embodiments wherein the subject-matter which "comprises" specifically listed elements does not comprise further elements as well as embodiments wherein the subject-matter which "comprises" specifically listed elements may and/or indeed does encompass further elements. Likewise, the expression "have" is to be understood as the expression "comprise", also including and specifically referring to the expressions "consist essentially of' and "consist of". The term "consist essentially of", where possible, in particular refers to embodiments wherein the subject-matter comprises 20% or less, in particular 15% or less, 10% or less or especially 5% or less further elements in addition to the specifically listed elements of which the subject-matter consists essentially of.

### SPECIFIC EMBODIMENTS

In the following, specific embodiments of the present invention are described.

Embodiment 1. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint.

Embodiment 2. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules.

Embodiment 3. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules, and
   wherein the portion of said plurality of partitions that contains zero target nucleic acid molecules is 0.2% or less, preferably 0.02% or less.

Embodiment 4. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules, and
   wherein the first determination timepoint is during or directly after any one of PCR cycles 10 to 35; preferably any one of PCR cycles 20 to 30, for example PCR cycle 25.

Embodiment 5. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules, and
   wherein the second determination timepoint is
      (i) during the plateau phase of the amplification; and/or
      (ii) during or directly after any one of PCR cycles 30 to 80; preferably any one of PCR cycles 45 to 55, for example PCR cycle 50.

Embodiment 6. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules, and
   wherein the second determination timepoint is
      (i) during the baseline phase of the amplification; and/or
      (ii) during or directly after any one of PCR cycles 1 to 20; preferably any one of PCR cycles 1 to 15, for example PCR cycle 1 or PCR cycle 5.

Embodiment 7. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
      wherein the detectable signal correlates to the amount of said target amplicons; and
      wherein the first determination timepoint and the second determination timepoint are separated by at least 3 PCR cycles.
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,

Embodiment 8. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity.

Embodiment 9. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity.

Embodiment 10. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein the calculation involves
      (i) calculating a relative value by dividing the intensity of the detectable signal determined at the first determination timepoint by the intensity of the detectable signal determined at the second determination timepoint; and
      (ii) comparing the relative value to reference relative values and/or a reference curve;
   wherein optionally the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons; and
   wherein optionally the reference nucleic acid molecules contain the same target nucleic acid sequence as the target nucleic acid molecules, and wherein the reference amplicons are produced by amplification of said target nucleic acid sequence present on the reference nucleic acid molecules; and
   wherein optionally the reference curve is a plot of the reference relative values of the at least two reference samples against the known number of reference nucleic acid molecules in the at least two reference samples.

Embodiment 11. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules, and
   wherein the method is simultaneously performed for two or more different target nucleic acid sequences; wherein for each different target nucleic acid sequence respective target amplicons are produced in step c), and for each different target amplicon a different detectable signal is used in step d) which correlates to the amount the respective target amplicon.

Embodiment 12 A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the portion of said plurality of partitions that contains zero target nucleic acid molecules is 0.2% or less, preferably 0.02% or less.

Embodiment 13. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the portion of said plurality of partitions that contains zero target nucleic acid molecules is 0.2% or less, preferably 0.02% or less, and
   wherein the first determination timepoint is during or directly after any one of PCR cycles 10 to 35; preferably any one of PCR cycles 20 to 30, for example PCR cycle 25.

Embodiment 14. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the portion of said plurality of partitions that contains zero target nucleic acid molecules is 0.2% or less, preferably 0.02% or less, and
   wherein the second determination timepoint is
      (i) during the plateau phase of the amplification; and/or
      (ii) during or directly after any one of PCR cycles 30 to 80 ; preferably any one of PCR cycles 45 to 55, for example PCR cycle 50.

Embodiment 15. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the portion of said plurality of partitions that contains zero target nucleic acid molecules is 0.2% or less, preferably 0.02% or less, and
   wherein the second determination timepoint is
      (i) during the baseline phase of the amplification; and/or
      (ii) during or directly after any one of PCR cycles 1 to 20; preferably any one of PCR cycles 1 to 15, for example PCR cycle 1 or PCR cycle 5.

Embodiment 16. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the portion of said plurality of partitions that contains zero target nucleic acid molecules is 0.2% or less, preferably 0.02% or less, and
   wherein the first determination timepoint and the second determination timepoint are separated by at least 3 PCR cycles.

Embodiment 17. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the portion of said plurality of partitions that contains zero target nucleic acid molecules is 0.2% or less, preferably 0.02% or less, and
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity.

Embodiment 18. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the portion of said plurality of partitions that contains zero target nucleic acid molecules is 0.2% or less, preferably 0.02% or less, and
   wherein the intensity of the detectable signal is only determined at the first determination timepoint and at the second determination timepoint, and optionally at a third determination timepoint during the baseline phase of the amplification.

Embodiment 19. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the portion of said plurality of partitions that contains zero target nucleic acid molecules is 0.2% or less, preferably 0.02% or less, and
   wherein the calculation involves
      (i) calculating a relative value by dividing the intensity of the detectable signal determined at the first determination timepoint by the intensity of the detectable signal determined at the second determination timepoint; and
      (ii) comparing the relative value to reference relative values and/or a reference curve;
   wherein optionally the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons; and
   wherein optionally the reference nucleic acid molecules contain the same target nucleic acid sequence as the target nucleic acid molecules, and wherein the reference amplicons are produced by amplification of said target nucleic acid sequence present on the reference nucleic acid molecules; and
   wherein optionally the reference curve is a plot of the reference relative values of the at least two reference samples against the known number of reference nucleic acid molecules in the at least two reference samples.

Embodiment 20. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the portion of said plurality of partitions that contains zero target nucleic acid molecules is 0.2% or less, preferably 0.02% or less, and
   wherein the method is simultaneously performed for two or more different target nucleic acid sequences; wherein for each different target nucleic acid sequence respective target amplicons are produced in step c), and for each different target amplicon a different detectable signal is used in step d) which correlates to the amount the respective target amplicon.

Embodiment 21. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the first determination timepoint is during or directly after any one of PCR cycles 10 to 35; preferably any one of PCR cycles 20 to 30, for example PCR cycle 25.

Embodiment 22. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the first determination timepoint is during or directly after any one of PCR cycles 10 to 35; preferably any one of PCR cycles 20 to 30, for example PCR cycle 25, and
   wherein the second determination timepoint is
      (i) during the plateau phase of the amplification; and/or
      (ii) during or directly after any one of PCR cycles 30 to 80; preferably any one of PCR cycles 45 to 55, for example PCR cycle 50.

Embodiment 23. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the first determination timepoint is during or directly after any one of PCR cycles 10 to 35; preferably any one of PCR cycles 20 to 30, for example PCR cycle 25, and
   wherein the second determination timepoint is
      (i) during the baseline phase of the amplification; and/or
      (ii) during or directly after any one of PCR cycles 1 to 20; preferably any one of PCR cycles 1 to 15, for example PCR cycle 1 or PCR cycle 5.

Embodiment 24. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the first determination timepoint is during or directly after any one of PCR cycles 10 to 35; preferably any one of PCR cycles 20 to 30, for example PCR cycle 25, and
   wherein the first determination timepoint and the second determination timepoint are separated by at least 3 PCR cycles.

Embodiment 25. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the first determination timepoint is during or directly after any one of PCR cycles 10 to 35; preferably any one of PCR cycles 20 to 30, for example PCR cycle 25, and
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity.

Embodiment 26. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the first determination timepoint is during or directly after any one of PCR cycles 10 to 35; preferably any one of PCR cycles 20 to 30, for example PCR cycle 25, and
   wherein the intensity of the detectable signal is only determined at the first determination timepoint and at the second determination timepoint, and optionally at a third determination timepoint during the baseline phase of the amplification.

Embodiment 27. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the first determination timepoint is during or directly after any one of PCR cycles 10 to 35; preferably any one of PCR cycles 20 to 30, for example PCR cycle 25, and
   wherein the calculation involves
      (i) calculating a relative value by dividing the intensity of the detectable signal determined at the first determination timepoint by the intensity of the detectable signal determined at the second determination timepoint; and
      (ii) comparing the relative value to reference relative values and/or a reference curve;
   wherein optionally the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons; and
   wherein optionally the reference nucleic acid molecules contain the same target nucleic acid sequence as the target nucleic acid molecules, and wherein the reference amplicons are produced by amplification of said target nucleic acid sequence present on the reference nucleic acid molecules; and
   wherein optionally the reference curve is a plot of the reference relative values of the at least two reference samples against the known number of reference nucleic acid molecules in the at least two reference samples.

Embodiment 28. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the first determination timepoint is during or directly after any one of PCR cycles 10 to 35; preferably any one of PCR cycles 20 to 30, for example PCR cycle 25, and
   wherein the method is simultaneously performed for two or more different target nucleic acid sequences; wherein for each different target nucleic acid sequence respective target amplicons are produced in step c), and for each different target amplicon a different detectable signal is used in step d) which correlates to the amount the respective target amplicon.

Embodiment 29. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the second determination timepoint is
   (i) during the plateau phase of the amplification; and/or
   (ii) during or directly after any one of PCR cycles 30 to 80 ; preferably any one of PCR cycles 45 to 55, for example PCR cycle 50.

Embodiment 30. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint, wherein the second determination timepoint is
   (i) during the plateau phase of the amplification; and/or
   (ii) during or directly after any one of PCR cycles 30 to 80 ; preferably any one of PCR cycles 45 to 55, for example PCR cycle 50,
   wherein the second determination timepoint is
   (i) during the baseline phase of the amplification; and/or
   (ii) during or directly after any one of PCR cycles 1 to 20; preferably any one of PCR cycles 1 to 15, for example PCR cycle 1 or PCR cycle 5.

Embodiment 31. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint, wherein the second determination timepoint is
   (i) during the plateau phase of the amplification; and/or
   (ii) during or directly after any one of PCR cycles 30 to 80 ; preferably any one of PCR cycles 45 to 55, for example PCR cycle 50,
   wherein the first determination timepoint and the second determination timepoint are separated by at least 3 PCR cycles.

Embodiment 32. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint, wherein the second determination timepoint is
   (i) during the plateau phase of the amplification; and/or
   (ii) during or directly after any one of PCR cycles 30 to 80 ; preferably any one of PCR cycles 45 to 55, for example PCR cycle 50,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity.

Embodiment 33. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint, wherein the second determination timepoint is
   (i) during the plateau phase of the amplification; and/or
   (ii) during or directly after any one of PCR cycles 30 to 80 ; preferably any one of PCR cycles 45 to 55, for example PCR cycle 50,
   wherein the intensity of the detectable signal is only determined at the first determination timepoint and at the second determination timepoint, and optionally at a third determination timepoint during the baseline phase of the amplification.

Embodiment 34. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint, wherein the second determination timepoint is
   (i) during the plateau phase of the amplification; and/or
   (ii) during or directly after any one of PCR cycles 30 to 80 ; preferably any one of PCR cycles 45 to 55, for example PCR cycle 50,

   wherein the calculation involves
      (i) calculating a relative value by dividing the intensity of the detectable signal determined at the first determination timepoint by the intensity of the detectable signal determined at the second determination timepoint; and
      (ii) comparing the relative value to reference relative values and/or a reference curve;
   wherein optionally the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons; and
   wherein optionally the reference nucleic acid molecules contain the same target nucleic acid sequence as the target nucleic acid molecules, and wherein the reference amplicons are produced by amplification of said target nucleic acid sequence present on the reference nucleic acid molecules; and
   wherein optionally the reference curve is a plot of the reference relative values of the at least two reference samples against the known number of reference nucleic acid molecules in the at least two reference samples.

Embodiment 35. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint, wherein the second determination timepoint is
   (i) during the plateau phase of the amplification; and/or
   (ii) during or directly after any one of PCR cycles 30 to 80 ; preferably any one of PCR cycles 45 to 55, for example PCR cycle 50,
   wherein the method is simultaneously performed for two or more different target nucleic acid sequences; wherein for each different target nucleic acid sequence respective target amplicons are produced in step c), and for each different target amplicon a different detectable signal is used in step d) which correlates to the amount the respective target amplicon.

Embodiment 36. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the second determination timepoint is
   (i) during the baseline phase of the amplification; and/or
   (ii) during or directly after any one of PCR cycles 1 to 20; preferably any one of PCR cycles 1 to 15, for example PCR cycle 1 or PCR cycle 5.

Embodiment 37. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the second determination timepoint is
      (i) during the baseline phase of the amplification; and/or
      (ii) during or directly after any one of PCR cycles 1 to 20; preferably any one of PCR cycles 1 to 15, for example PCR cycle 1 or PCR cycle 5,
   wherein the first determination timepoint and the second determination timepoint are separated by at least 3 PCR cycles.

Embodiment 38. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the second determination timepoint is
      (i) during the baseline phase of the amplification; and/or
      (ii) during or directly after any one of PCR cycles 1 to 20; preferably any one of PCR cycles 1 to 15, for example PCR cycle 1 or PCR cycle 5,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity.

Embodiment 39. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the second determination timepoint is
      (i) during the baseline phase of the amplification; and/or
      (ii) during or directly after any one of PCR cycles 1 to 20; preferably any one of PCR cycles 1 to 15, for example PCR cycle 1 or PCR cycle 5,
   wherein the intensity of the detectable signal is only determined at the first determination timepoint and at the second determination timepoint, and optionally at a third determination timepoint during the baseline phase of the amplification.

Embodiment 40. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the second determination timepoint is
      (i) during the baseline phase of the amplification; and/or
      (ii) during or directly after any one of PCR cycles 1 to 20; preferably any one of PCR cycles 1 to 15, for example PCR cycle 1 or PCR cycle 5,
   wherein the calculation involves
      (i) calculating a relative value by dividing the intensity of the detectable signal determined at the first determination timepoint by the intensity of the detectable signal determined at the second determination timepoint; and
      (ii) comparing the relative value to reference relative values and/or a reference curve;
   wherein optionally the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons; and
   wherein optionally the reference nucleic acid molecules contain the same target nucleic acid sequence as the target nucleic acid molecules, and wherein the reference amplicons are produced by amplification of said target nucleic acid sequence present on the reference nucleic acid molecules; and
   wherein optionally the reference curve is a plot of the reference relative values of the at least two reference samples against the known number of reference nucleic acid molecules in the at least two reference samples.

Embodiment 41. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the second determination timepoint is
      (i) during the baseline phase of the amplification; and/or
      (ii) during or directly after any one of PCR cycles 1 to 20; preferably any one of PCR cycles 1 to 15, for example PCR cycle 1 or PCR cycle 5,
   wherein the method is simultaneously performed for two or more different target nucleic acid sequences; wherein for each different target nucleic acid sequence respective target amplicons are produced in step c), and for each different target amplicon a different detectable signal is used in step d) which correlates to the amount the respective target amplicon.

Embodiment 42. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the first determination timepoint and the second determination timepoint are separated by at least 3 PCR cycles.

Embodiment 43. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the first determination timepoint and the second determination timepoint are separated by at least 3 PCR cycles, and
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity.

Embodiment 44. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the first determination timepoint and the second determination timepoint are separated by at least 3 PCR cycles, and
   wherein the intensity of the detectable signal is only determined at the first determination timepoint and at the second determination timepoint, and optionally at a third determination timepoint during the baseline phase of the amplification.

Embodiment 45. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the first determination timepoint and the second determination timepoint are separated by at least 3 PCR cycles, and
   wherein the calculation involves
      (i) calculating a relative value by dividing the intensity of the detectable signal determined at the first determination timepoint by the intensity of the detectable signal determined at the second determination timepoint; and
      (ii) comparing the relative value to reference relative values and/or a reference curve;
   wherein optionally the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons; and
   wherein optionally the reference nucleic acid molecules contain the same target nucleic acid sequence as the target nucleic acid molecules, and wherein the reference amplicons are produced by amplification of said target nucleic acid sequence present on the reference nucleic acid molecules; and
   wherein optionally the reference curve is a plot of the reference relative values of the at least two reference samples against the known number of reference nucleic acid molecules in the at least two reference samples.

Embodiment 46. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the first determination timepoint and the second determination timepoint are separated by at least 3 PCR cycles, and
   wherein the method is simultaneously performed for two or more different target nucleic acid sequences; wherein for each different target nucleic acid sequence respective target amplicons are produced in step c), and for each different target amplicon a different detectable signal is used in step d) which correlates to the amount the respective target amplicon.

Embodiment 47. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity.

Embodiment 48. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or
   wherein the individual intensities of the partitions are used to calculate a mean or median intensity,
   wherein the intensity of the detectable signal is only determined at the first determination timepoint and at the second determination timepoint, and optionally at a third determination timepoint during the baseline phase of the amplification.

Embodiment 49. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity,
   wherein the calculation involves
      (i) calculating a relative value by dividing the intensity of the detectable signal determined at the first determination timepoint by the intensity of the detectable signal determined at the second determination timepoint; and
      (ii) comparing the relative value to reference relative values and/or a reference curve;
   wherein optionally the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons; and
   wherein optionally the reference nucleic acid molecules contain the same target nucleic acid sequence as the target nucleic acid molecules, and wherein the reference amplicons are produced by amplification of said target nucleic acid sequence present on the reference nucleic acid molecules; and
   wherein optionally the reference curve is a plot of the reference relative values of the at least two reference samples against the known number of reference nucleic acid molecules in the at least two reference samples.

Embodiment 50. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity,
   wherein the method is simultaneously performed for two or more different target nucleic acid sequences; wherein for each different target nucleic acid sequence respective target amplicons are produced in step c), and for each different target amplicon a different detectable signal is used in step d) which correlates to the amount the respective target amplicon.

Embodiment 51. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the intensity of the detectable signal is only determined at the first determination timepoint and at the second determination timepoint, and optionally at a third determination timepoint during the baseline phase of the amplification.

Embodiment 52. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the intensity of the detectable signal is only determined at the first determination timepoint and at the second determination timepoint, and optionally at a third determination timepoint during the baseline phase of the amplification, and
   wherein the calculation involves
      (i) calculating a relative value by dividing the intensity of the detectable signal determined at the first determination timepoint by the intensity of the detectable signal determined at the second determination timepoint; and
      (ii) comparing the relative value to reference relative values and/or a reference curve;
   wherein optionally the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons; and
   wherein optionally the reference nucleic acid molecules contain the same target nucleic acid sequence as the target nucleic acid molecules, and wherein the reference amplicons are produced by amplification of said target nucleic acid sequence present on the reference nucleic acid molecules; and
   wherein optionally the reference curve is a plot of the reference relative values of the at least two reference samples against the known number of reference nucleic acid molecules in the at least two reference samples.

Embodiment 53. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the intensity of the detectable signal is only determined at the first determination timepoint and at the second determination timepoint, and optionally at a third determination timepoint during the baseline phase of the amplification, and
   wherein the method is simultaneously performed for two or more different target nucleic acid sequences; wherein for each different target nucleic acid sequence respective target amplicons are produced in step c), and for each different target amplicon a different detectable signal is used in step d) which correlates to the amount the respective target amplicon.

Embodiment 54. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the calculation involves
      (i) calculating a relative value by dividing the intensity of the detectable signal determined at the first determination timepoint by the intensity of the detectable signal determined at the second determination timepoint; and
      (ii) comparing the relative value to reference relative values and/or a reference curve;
   wherein optionally the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons; and
      wherein optionally the reference nucleic acid molecules contain the same target nucleic acid sequence as the target nucleic acid molecules, and wherein the reference amplicons are produced by amplification of said target nucleic acid sequence present on the reference nucleic acid molecules; and
      wherein optionally the reference curve is a plot of the reference relative values of the at least two reference samples against the known number of reference nucleic acid molecules in the at least two reference samples.

Embodiment 55. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the calculation involves
      (i) calculating a relative value by dividing the intensity of the detectable signal determined at the first determination timepoint by the intensity of the detectable signal determined at the second determination timepoint; and
      (ii) comparing the relative value to reference relative values and/or a reference curve;
   wherein optionally the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons; and
   wherein optionally the reference nucleic acid molecules contain the same target nucleic acid sequence as the target nucleic acid molecules, and wherein the reference amplicons are produced by amplification of said target nucleic acid sequence present on the reference nucleic acid molecules; and
   wherein optionally the reference curve is a plot of the reference relative values of the at least two reference samples against the known number of reference nucleic acid molecules in the at least two reference samples,
   wherein the method is simultaneously performed for two or more different target nucleic acid sequences; wherein for each different target nucleic acid sequence respective target amplicons are produced in step c), and for each different target amplicon a different detectable signal is used in step d) which correlates to the amount the respective target amplicon.

Embodiment 56. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the method is simultaneously performed for two or more different target nucleic acid sequences; wherein for each different target nucleic acid sequence respective target amplicons are produced in step c), and for each different target amplicon a different detectable signal is used in step d) which correlates to the amount the respective target amplicon.

Embodiment 57. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein the portion of said plurality of partitions that contains zero target nucleic acid molecules is 0.2% or less, preferably 0.02% or less.
   wherein the first determination timepoint is during or directly after any one of PCR cycles 10 to 35; preferably any one of PCR cycles 20 to 30, for example PCR cycle 25.

Embodiment 58. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein the portion of said plurality of partitions that contains zero target nucleic acid molecules is 0.2% or less, preferably 0.02% or less.
   wherein the first determination timepoint is during or directly after any one of PCR cycles 10 to 35; preferably any one of PCR cycles 20 to 30, for example PCR cycle 25,
   wherein the second determination timepoint is
      (i) during the plateau phase of the amplification; and/or
      (ii) during or directly after any one of PCR cycles 30 to 80 ; preferably any one of PCR cycles 45 to 55, for example PCR cycle 50.

Embodiment 59. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein the portion of said plurality of partitions that contains zero target nucleic acid molecules is 0.2% or less, preferably 0.02% or less.
   wherein the first determination timepoint is during or directly after any one of PCR cycles 10 to 35; preferably any one of PCR cycles 20 to 30, for example PCR cycle 25,
   wherein the second determination timepoint is
      (i) during the baseline phase of the amplification; and/or
      (ii) during or directly after any one of PCR cycles 1 to 20; preferably any one of PCR cycles 1 to 15, for example PCR cycle 1 or PCR cycle 5.

Embodiment 60. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein the portion of said plurality of partitions that contains zero target nucleic acid molecules is 0.2% or less, preferably 0.02% or less.
   wherein the first determination timepoint is during or directly after any one of PCR cycles 10 to 35; preferably any one of PCR cycles 20 to 30, for example PCR cycle 25,
   wherein the second determination timepoint is
      (i) during the plateau phase of the amplification; and/or
      (ii) during or directly after any one of PCR cycles 30 to 80 ; preferably any one of PCR cycles 45 to 55, for example PCR cycle 50,
   wherein the first determination timepoint and the second determination timepoint are separated by at least 3 PCR cycles.

Embodiment 61. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein the portion of said plurality of partitions that contains zero target nucleic acid molecules is 0.2% or less, preferably 0.02% or less.
   wherein the first determination timepoint is during or directly after any one of PCR cycles 10 to 35; preferably any one of PCR cycles 20 to 30, for example PCR cycle 25,
   wherein the second determination timepoint is
      (i) during the baseline phase of the amplification; and/or
      (ii) during or directly after any one of PCR cycles 1 to 20; preferably any one of PCR cycles 1 to 15, for example PCR cycle 1 or PCR cycle 5,
   wherein the first determination timepoint and the second determination timepoint are separated by at least 3 PCR cycles.

Embodiment 62. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein the portion of said plurality of partitions that contains zero target nucleic acid molecules is 0.2% or less, preferably 0.02% or less.
   wherein the first determination timepoint is during or directly after any one of PCR cycles 10 to 35; preferably any one of PCR cycles 20 to 30, for example PCR cycle 25,
   wherein the second determination timepoint is
      (i) during the plateau phase of the amplification; and/or
      (ii) during or directly after any one of PCR cycles 30 to 80 ; preferably any one of PCR cycles 45 to 55, for example PCR cycle 50,
   wherein the first determination timepoint and the second determination timepoint are separated by at least 3 PCR cycles,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity.

Embodiment 63. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein the portion of said plurality of partitions that contains zero target nucleic acid molecules is 0.2% or less, preferably 0.02% or less.
   wherein the first determination timepoint is during or directly after any one of PCR cycles 10 to 35; preferably any one of PCR cycles 20 to 30, for example PCR cycle 25,
   wherein the second determination timepoint is
      (i) during the baseline phase of the amplification; and/or
      (ii) during or directly after any one of PCR cycles 1 to 20; preferably any one of PCR cycles 1 to 15, for example PCR cycle 1 or PCR cycle 5,
   wherein the first determination timepoint and the second determination timepoint are separated by at least 3 PCR cycles.
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity.

Embodiment 64. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein the portion of said plurality of partitions that contains zero target nucleic acid molecules is 0.2% or less, preferably 0.02% or less.
   wherein the first determination timepoint is during or directly after any one of PCR cycles 10 to 35; preferably any one of PCR cycles 20 to 30, for example PCR cycle 25,
   wherein the second determination timepoint is
      (i) during the plateau phase of the amplification; and/or
      (ii) during or directly after any one of PCR cycles 30 to 80 ; preferably any one of PCR cycles 45 to 55, for example PCR cycle 50,
   wherein the first determination timepoint and the second determination timepoint are separated by at least 3 PCR cycles,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity,
   wherein the intensity of the detectable signal is only determined at the first determination timepoint and at the second determination timepoint, and optionally at a third determination timepoint during the baseline phase of the amplification.

Embodiment 65. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein the portion of said plurality of partitions that contains zero target nucleic acid molecules is 0.2% or less, preferably 0.02% or less.
   wherein the first determination timepoint is during or directly after any one of PCR cycles 10 to 35; preferably any one of PCR cycles 20 to 30, for example PCR cycle 25,
   wherein the second determination timepoint is
      (i) during the baseline phase of the amplification; and/or
      (ii) during or directly after any one of PCR cycles 1 to 20; preferably any one of PCR cycles 1 to 15, for example PCR cycle 1 or PCR cycle 5,
   wherein the first determination timepoint and the second determination timepoint are separated by at least 3 PCR cycles.
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity,
   wherein the intensity of the detectable signal is only determined at the first determination timepoint and at the second determination timepoint, and optionally at a third determination timepoint during the baseline phase of the amplification.

Embodiment 66. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein the portion of said plurality of partitions that contains zero target nucleic acid molecules is 0.2% or less, preferably 0.02% or less.
   wherein the first determination timepoint is during or directly after any one of PCR cycles 10 to 35; preferably any one of PCR cycles 20 to 30, for example PCR cycle 25,
   wherein the second determination timepoint is
      (i) during the plateau phase of the amplification; and/or
      (ii) during or directly after any one of PCR cycles 30 to 80 ; preferably any one of PCR cycles 45 to 55, for example PCR cycle 50,
   wherein the first determination timepoint and the second determination timepoint are separated by at least 3 PCR cycles,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity,
   wherein the intensity of the detectable signal is only determined at the first determination timepoint and at the second determination timepoint, and optionally at a third determination timepoint during the baseline phase of the amplification,
   wherein the calculation involves
      (i) calculating a relative value by dividing the intensity of the detectable signal determined at the first determination timepoint by the intensity of the detectable signal determined at the second determination timepoint; and
      (ii) comparing the relative value to reference relative values and/or a reference curve;
   wherein optionally the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons; and
   wherein optionally the reference nucleic acid molecules contain the same target nucleic acid sequence as the target nucleic acid molecules, and wherein the reference amplicons are produced by amplification of said target nucleic acid sequence present on the reference nucleic acid molecules; and
   wherein optionally the reference curve is a plot of the reference relative values of the at least two reference samples against the known number of reference nucleic acid molecules in the at least two reference samples.

Embodiment 67. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein the portion of said plurality of partitions that contains zero target nucleic acid molecules is 0.2% or less, preferably 0.02% or less.
   wherein the first determination timepoint is during or directly after any one of PCR cycles 10 to 35; preferably any one of PCR cycles 20 to 30, for example PCR cycle 25,
   wherein the second determination timepoint is
      (i) during the baseline phase of the amplification; and/or
      (ii) during or directly after any one of PCR cycles 1 to 20; preferably any one of PCR cycles 1 to 15, for example PCR cycle 1 or PCR cycle 5,
   wherein the first determination timepoint and the second determination timepoint are separated by at least 3 PCR cycles.
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity,
   wherein the intensity of the detectable signal is only determined at the first determination timepoint and at the second determination timepoint, and optionally at a third determination timepoint during the baseline phase of the amplification,
   wherein the calculation involves
      (i) calculating a relative value by dividing the intensity of the detectable signal determined at the first determination timepoint by the intensity of the detectable signal determined at the second determination timepoint; and
      (ii) comparing the relative value to reference relative values and/or a reference curve;
   wherein optionally the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons; and
   wherein optionally the reference nucleic acid molecules contain the same target nucleic acid sequence as the target nucleic acid molecules, and wherein the reference amplicons are produced by amplification of said target nucleic acid sequence present on the reference nucleic acid molecules; and
   wherein optionally the reference curve is a plot of the reference relative values of the at least two reference samples against the known number of reference nucleic acid molecules in the at least two reference samples.

Embodiment 68. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein the portion of said plurality of partitions that contains zero target nucleic acid molecules is 0.2% or less, preferably 0.02% or less.
   wherein the first determination timepoint is during or directly after any one of PCR cycles 10 to 35; preferably any one of PCR cycles 20 to 30, for example PCR cycle 25,
   wherein the second determination timepoint is
      (i) during the plateau phase of the amplification; and/or
      (ii) during or directly after any one of PCR cycles 30 to 80 ; preferably any one of PCR cycles 45 to 55, for example PCR cycle 50,
   wherein the first determination timepoint and the second determination timepoint are separated by at least 3 PCR cycles,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity,
   wherein the intensity of the detectable signal is only determined at the first determination timepoint and at the second determination timepoint, and optionally at a third determination timepoint during the baseline phase of the amplification,
   wherein the calculation involves
      (i) calculating a relative value by dividing the intensity of the detectable signal determined at the first determination timepoint by the intensity of the detectable signal determined at the second determination timepoint; and
      (ii) comparing the relative value to reference relative values and/or a reference curve;
   wherein optionally the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons; and
   wherein optionally the reference nucleic acid molecules contain the same target nucleic acid sequence as the target nucleic acid molecules, and wherein the reference amplicons are produced by amplification of said target nucleic acid sequence present on the reference nucleic acid molecules; and
   wherein optionally the reference curve is a plot of the reference relative values of the at least two reference samples against the known number of reference nucleic acid molecules in the at least two reference samples,
   wherein the method is simultaneously performed for two or more different target nucleic acid sequences; wherein for each different target nucleic acid sequence respective target amplicons are produced in step c), and for each different target amplicon a different detectable signal is used in step d) which correlates to the amount the respective target amplicon.

Embodiment 69. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein the portion of said plurality of partitions that contains zero target nucleic acid molecules is 0.2% or less, preferably 0.02% or less.
   wherein the first determination timepoint is during or directly after any one of PCR cycles 10 to 35; preferably any one of PCR cycles 20 to 30, for example PCR cycle 25,
   wherein the second determination timepoint is
      (i) during the baseline phase of the amplification; and/or
      (ii) during or directly after any one of PCR cycles 1 to 20; preferably any one of PCR cycles 1 to 15, for example PCR cycle 1 or PCR cycle 5,
   wherein the first determination timepoint and the second determination timepoint are separated by at least 3 PCR cycles.
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity,
   wherein the intensity of the detectable signal is only determined at the first determination timepoint and at the second determination timepoint, and optionally at a third determination timepoint during the baseline phase of the amplification,
   wherein the calculation involves
      (i) calculating a relative value by dividing the intensity of the detectable signal determined at the first determination timepoint by the intensity of the detectable signal determined at the second determination timepoint; and
      (ii) comparing the relative value to reference relative values and/or a reference curve;
   wherein optionally the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons; and
   wherein optionally the reference nucleic acid molecules contain the same target nucleic acid sequence as the target nucleic acid molecules, and wherein the reference amplicons are produced by amplification of said target nucleic acid sequence present on the reference nucleic acid molecules; and
   wherein optionally the reference curve is a plot of the reference relative values of the at least two reference samples against the known number of reference nucleic acid molecules in the at least two reference samples,
   wherein the method is simultaneously performed for two or more different target nucleic acid sequences; wherein for each different target nucleic acid sequence respective target amplicons are produced in step c), and for each different target amplicon a different detectable signal is used in step d) which correlates to the amount the respective target amplicon.

Embodiment 70. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein the portion of said plurality of partitions that contains zero target nucleic acid molecules is 0.2% or less, preferably 0.02% or less,
   wherein the first determination timepoint and the second determination timepoint are separated by at least 3 PCR cycles.

Embodiment 71. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein the portion of said plurality of partitions that contains zero target nucleic acid molecules is 0.2% or less, preferably 0.02% or less,
   wherein the first determination timepoint and the second determination timepoint are separated by at least 3 PCR cycles,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity.

Embodiment 72. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein the portion of said plurality of partitions that contains zero target nucleic acid molecules is 0.2% or less, preferably 0.02% or less,
   wherein the first determination timepoint and the second determination timepoint are separated by at least 3 PCR cycles,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity,
   wherein the intensity of the detectable signal is only determined at the first determination timepoint and at the second determination timepoint, and optionally at a third determination timepoint during the baseline phase of the amplification.

Embodiment 73. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein the portion of said plurality of partitions that contains zero target nucleic acid molecules is 0.2% or less, preferably 0.02% or less,
   wherein the first determination timepoint and the second determination timepoint are separated by at least 3 PCR cycles,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity,
   wherein the intensity of the detectable signal is only determined at the first determination timepoint and at the second determination timepoint, and optionally at a third determination timepoint during the baseline phase of the amplification,
   wherein the calculation involves
      (i) calculating a relative value by dividing the intensity of the detectable signal determined at the first determination timepoint by the intensity of the detectable signal determined at the second determination timepoint; and
      (ii) comparing the relative value to reference relative values and/or a reference curve;
   wherein optionally the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons; and
   wherein optionally the reference nucleic acid molecules contain the same target nucleic acid sequence as the target nucleic acid molecules, and wherein the reference amplicons are produced by amplification of said target nucleic acid sequence present on the reference nucleic acid molecules; and
   wherein optionally the reference curve is a plot of the reference relative values of the at least two reference samples against the known number of reference nucleic acid molecules in the at least two reference samples.

Embodiment 74. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein the portion of said plurality of partitions that contains zero target nucleic acid molecules is 0.2% or less, preferably 0.02% or less,
   wherein the first determination timepoint and the second determination timepoint are separated by at least 3 PCR cycles,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity,
   wherein the intensity of the detectable signal is only determined at the first determination timepoint and at the second determination timepoint, and optionally at a third determination timepoint during the baseline phase of the amplification,
   wherein the calculation involves
      (i) calculating a relative value by dividing the intensity of the detectable signal determined at the first determination timepoint by the intensity of the detectable signal determined at the second determination timepoint; and
      (ii) comparing the relative value to reference relative values and/or a reference curve;
   wherein optionally the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons; and
   wherein optionally the reference nucleic acid molecules contain the same target nucleic acid sequence as the target nucleic acid molecules, and wherein the reference amplicons are produced by amplification of said target nucleic acid sequence present on the reference nucleic acid molecules; and
   wherein optionally the reference curve is a plot of the reference relative values of the at least two reference samples against the known number of reference nucleic acid molecules in the at least two reference samples,
   wherein the method is simultaneously performed for two or more different target nucleic acid sequences; wherein for each different target nucleic acid sequence respective target amplicons are produced in step c), and for each different target amplicon a different detectable signal is used in step d) which correlates to the amount the respective target amplicon.

Embodiment 75. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein the portion of said plurality of partitions that contains zero target nucleic acid molecules is 0.2% or less, preferably 0.02% or less,
   wherein the method is simultaneously performed for two or more different target nucleic acid sequences; wherein for each different target nucleic acid sequence respective target amplicons are produced in step c), and for each different target amplicon a different detectable signal is used in step d) which correlates to the amount the respective target amplicon.

Embodiment 76. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein each of said partitions in step b) contains on average more than one target nucleic acid molecules, especially on average five or more target nucleic acid molecules,
   wherein the portion of said plurality of partitions that contains zero target nucleic acid molecules is 0.2% or less, preferably 0.02% or less,
   wherein the first determination timepoint and the second determination timepoint are separated by at least 3 PCR cycles,
   wherein the method is simultaneously performed for two or more different target nucleic acid sequences; wherein for each different target nucleic acid sequence respective target amplicons are produced in step c), and for each different target amplicon a different detectable signal is used in step d) which correlates to the amount the respective target amplicon.

Embodiment 77. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the first determination timepoint and the second determination timepoint are separated by at least 3 PCR cycles,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity,
   wherein the intensity of the detectable signal is only determined at the first determination timepoint and at the second determination timepoint, and optionally at a third determination timepoint during the baseline phase of the amplification.

Embodiment 78. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the first determination timepoint and the second determination timepoint are separated by at least 3 PCR cycles,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity,
   wherein the intensity of the detectable signal is only determined at the first determination timepoint and at the second determination timepoint, and optionally at a third determination timepoint during the baseline phase of the amplification,
   wherein the method is simultaneously performed for two or more different target nucleic acid sequences; wherein for each different target nucleic acid sequence respective target amplicons are produced in step c), and for each different target amplicon a different detectable signal is used in step d) which correlates to the amount the respective target amplicon.

Embodiment 79. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the first determination timepoint and the second determination timepoint are separated by at least 3 PCR cycles,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity,
   wherein the intensity of the detectable signal is only determined at the first determination timepoint and at the second determination timepoint, and optionally at a third determination timepoint during the baseline phase of the amplification,
   wherein the calculation involves
      (i) calculating a relative value by dividing the intensity of the detectable signal determined at the first determination timepoint by the intensity of the detectable signal determined at the second determination timepoint; and
      (ii) comparing the relative value to reference relative values and/or a reference curve;
   wherein optionally the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons; and
   wherein optionally the reference nucleic acid molecules contain the same target nucleic acid sequence as the target nucleic acid molecules, and wherein the reference amplicons are produced by amplification of said target nucleic acid sequence present on the reference nucleic acid molecules; and
   wherein optionally the reference curve is a plot of the reference relative values of the at least two reference samples against the known number of reference nucleic acid molecules in the at least two reference samples.

Embodiment 80. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein the first determination timepoint and the second determination timepoint are separated by at least 3 PCR cycles,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity,
   wherein the intensity of the detectable signal is only determined at the first determination timepoint and at the second determination timepoint, and optionally at a third determination timepoint during the baseline phase of the amplification,
   wherein the calculation involves
      (i) calculating a relative value by dividing the intensity of the detectable signal determined at the first determination timepoint by the intensity of the detectable signal determined at the second determination timepoint; and
      (ii) comparing the relative value to reference relative values and/or a reference curve;
   wherein optionally the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons; and
      wherein optionally the reference nucleic acid molecules contain the same target nucleic acid sequence as the target nucleic acid molecules, and wherein the reference amplicons are produced by amplification of said target nucleic acid sequence present on the reference nucleic acid molecules; and
      wherein optionally the reference curve is a plot of the reference relative values of the at least two reference samples against the known number of reference nucleic acid molecules in the at least two reference samples,
      wherein the method is simultaneously performed for two or more different target nucleic acid sequences; wherein for each different target nucleic acid sequence respective target amplicons are produced in step c), and for each different target amplicon a different detectable signal is used in step d) which correlates to the amount the respective target amplicon.

Embodiment 81. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity,
   wherein the intensity of the detectable signal is only determined at the first determination timepoint and at the second determination timepoint, and optionally at a third determination timepoint during the baseline phase of the amplification,
   wherein the calculation involves
      (i) calculating a relative value by dividing the intensity of the detectable signal determined at the first determination timepoint by the intensity of the detectable signal determined at the second determination timepoint; and
      (ii) comparing the relative value to reference relative values and/or a reference curve;
   wherein optionally the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons; and
   wherein optionally the reference nucleic acid molecules contain the same target nucleic acid sequence as the target nucleic acid molecules, and wherein the reference amplicons are produced by amplification of said target nucleic acid sequence present on the reference nucleic acid molecules; and
   wherein optionally the reference curve is a plot of the reference relative values of the at least two reference samples against the known number of reference nucleic acid molecules in the at least two reference samples.

Embodiment 82. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
   (i) at a first determination timepoint during the exponential phase of the amplification, and
   (ii) at a second determination timepoint during a stationary phase of the amplification,
   wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint,
   wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or
   wherein the individual intensities of the partitions are used to calculate a mean or median intensity,
   wherein the intensity of the detectable signal is only determined at the first determination timepoint and at the second determination timepoint, and optionally at a third determination timepoint during the baseline phase of the amplification,
   wherein the calculation involves
      (i) calculating a relative value by dividing the intensity of the detectable signal determined at the first determination timepoint by the intensity of the detectable signal determined at the second determination timepoint; and
      (ii) comparing the relative value to reference relative values and/or a reference curve;
   wherein optionally the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons; and
   wherein optionally the reference nucleic acid molecules contain the same target nucleic acid sequence as the target nucleic acid molecules, and wherein the reference amplicons are produced by amplification of said target nucleic acid sequence present on the reference nucleic acid molecules; and
   wherein optionally the reference curve is a plot of the reference relative values of the at least two reference samples against the known number of reference nucleic acid molecules in the at least two reference samples,
   wherein the method is simultaneously performed for two or more different target nucleic acid sequences; wherein for each different target nucleic acid sequence respective target amplicons are produced in step c), and for each different target amplicon a different detectable signal is used in step d) which correlates to the amount the respective target amplicon.

Embodiment 83. The method according to any one of embodiments 1 to 82, wherein the partitions are spatially isolated partitions.

Embodiment 84. The method according to any one of embodiments 1 or 83, wherein each of said partitions in step b) contains on average more than one target nucleic acid molecules.

Embodiment 85. The method according to any one of embodiments 1 to 84, wherein each of said partitions in step b) contains on average five or more target nucleic acid molecules.

Embodiment 86. The method according to any one of embodiments 1 to 85, wherein the portion of said plurality of partitions that contains zero target nucleic acid molecules is insufficient in number to allow application of Poisson statistics.

Embodiment 87. The method according to any one of embodiments 1 to 86, wherein the portion of said plurality of partitions that contains zero target nucleic acid molecules is 0.2% or less, preferably 0.02% or less.

Embodiment 88. The method according to any one of embodiments 1 to 87, wherein each of said partitions when being subjected to the initial PCR cycle comprises amplification reagents sufficient to perform a polymerase chain reaction.

Embodiment 89. The method according to any one of embodiments 1 to 88, wherein each of said partitions when being subjected to the initial PCR cycle comprises detection reagents which produce the detectable signal.

Embodiment 90. The method according to embodiment 89, wherein the detection reagent comprises a fluorescent label and the detectable signal is a fluorescence signal.

Embodiment 91. The method according to any one of embodiments 1 to 90, wherein the first determination timepoint is during or directly after any one of PCR cycles 10 to 35; preferably any one of PCR cycles 20 to 30, for example PCR cycle 25.

Embodiment 92. The method according to any one of embodiments 1 to 91, wherein the second determination timepoint is during the plateau phase of the amplification.

Embodiment 93. The method according to any one of embodiments 1 to 92, wherein the second determination timepoint is during or directly after any one of PCR cycles 30 to 80; preferably any one of PCR cycles 45 to 55, for example PCR cycle 50.

Embodiment 94. The method according to any one of embodiments 1 to 91, wherein the second determination timepoint is during the baseline phase of the amplification.

Embodiment 95. The method according to any one of embodiments 1 to 91 and 94, wherein the second determination timepoint is during or directly after any one of PCR cycles 1 to 20; preferably any one of PCR cycles 1 to 15, for example PCR cycle 1 or PCR cycle 5.

Embodiment 96. The method according to any one of embodiments 1 to 95, wherein the first determination timepoint and the second determination timepoint are separated by at least 5 PCR cycles.

Embodiment 96. The method according to any one of embodiments 1 to 95, wherein the first determination timepoint and the second determination timepoint are separated by at least 10 PCR cycles.

Embodiment 97. The method according to any one of embodiments 1 to 96, wherein in the exponential phase the intensity of the detectable signal is significantly different from the intensity of the detectable signal during the stationary phases.

Embodiment 98. The method according to any one of embodiments 1 to 97, wherein in the exponential phase the amount of target amplicons is about 10% to about 90% of the amount of target amplicons at the end of the last PCR cycle.

Embodiment 99. The method according to any one of embodiments 1 to 98, wherein the first determination timepoint and/or the second determination timepoint are determined by one or more test runs.

Embodiment 100. The method according to any one of embodiments 1 to 99, wherein in step d) a common intensity of the detectable signal is determined for all partitions of the plurality of partitions, or wherein individual intensities of the detectable signal are measured for each partition of the plurality of partitions.

Embodiment 101. The method according to embodiment 100, wherein the individual intensities of the partitions are added up to provide a total intensity, or wherein the individual intensities of the partitions are used to calculate a mean or median intensity.

Embodiment 102. The method according to any one of embodiments 1 to 101, wherein the intensity of the detectable signal is only determined at the first determination timepoint and at the second determination timepoint, and optionally at a third determination timepoint during the baseline phase of the amplification.

Embodiment 103. The method according to any one of embodiments 1 to 101, wherein the intensity of the detectable signal is determined at one or more further determination timepoints different from the first and second determination timepoint.

Embodiment 104. The method according to embodiment 103, wherein one or more of the further determination timepoints are during the exponential phase of the amplification.

Embodiment 105. The method according to any one of embodiments 1 to 104, wherein the intensities of the detectable signal determined at the first determination timepoint and at the second determination timepoint are corrected by the intensity of the detectable signal determined at a third determination timepoint during the baseline phase of the amplification.

Embodiment 106. The method according to any one of embodiments 1 to 105, wherein the calculation involves a relative value obtained by dividing the intensity of the detectable signal determined at the first determination timepoint by the intensity of the detectable signal determined at the second determination timepoint.

Embodiment 107. The method according to embodiment 106, wherein calculating the initial number of target nucleic acid molecules which contain said target nucleic acid sequence comprises comparing the relative value to reference relative values and/or a reference curve.

Embodiment 108. The method according to embodiment 107, wherein the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons.

Embodiment 109. The method according to embodiment 108, wherein the reference nucleic acid molecules contain the same target nucleic acid sequence as the target nucleic acid molecules, and wherein the reference amplicons are produced by amplification of said target nucleic acid sequence present on the reference nucleic acid molecules.

Embodiment 110. The method according to embodiment 108 or 109, wherein the reference curve is a plot of the reference relative values of the at least two reference samples against the known number of reference nucleic acid molecules in the at least two reference samples.

Embodiment 111. A method of quantitating first target nucleic acid molecules and second target nucleic acid molecules in a sample comprising:
a) providing a sample comprising first target nucleic acid molecules which contain a first target nucleic acid sequence, and second target nucleic acid molecules which contain a second target nucleic acid sequence which is different from the first target nucleic acid sequence; and
b) separating said sample into a plurality of partitions; and
c) subjecting said plurality of partitions to multiple cycles of polymerase chain reaction (PCR cycles) for amplification of said first target nucleic acid sequence to produce first target amplicons in one or more of said partitions, and for amplification of said second target nucleic acid sequence to produce second target amplicons in one or more of said partitions; and
d) determining the intensity of a first detectable signal in said plurality of partitions
   (i) at a first determination timepoint during the exponential phase of the amplification, and
   (ii) at a second determination timepoint during a stationary phase of the amplification,

   wherein the first detectable signal correlates to the amount of said first target amplicons; and
   determining the number of partitions which are positive for said second target amplicons, wherein a positive partition has an intensity of a second detectable signal at a determination timepoint during the plateau phase of the amplification which is above a threshold value, wherein the second detectable signal correlates to the amount of said second target amplicons; and
e) calculating an initial number of first target nucleic acid molecules which contain said first target nucleic acid sequence that are present in said sample before said sample has been separated into said plurality of partitions, wherein said calculation is based on the relation between the intensity of the first detectable signal determined at the first determination timepoint and the intensity of the first detectable signal determined at the second determination timepoint; and
   calculating an initial number of second target nucleic acid molecules which contain said second target nucleic acid sequence that are present in said sample before said sample has been separated into said plurality of partitions based on the number or ratio of positive partitions.

Embodiment 112. A method of quantitating first target nucleic acid molecules and second target nucleic acid molecules in a sample comprising:
a) providing a sample comprising first target nucleic acid molecules which contain a first target nucleic acid sequence, and second target nucleic acid molecules which contain a second target nucleic acid sequence which is different from the first target nucleic acid sequence; and
b) separating said sample into a plurality of partitions; and
c) subjecting said plurality of partitions to multiple cycles of polymerase chain reaction (PCR cycles) for amplification of said first target nucleic acid sequence to produce first target amplicons in one or more of said partitions, and for amplification of said second target nucleic acid sequence to produce second target amplicons in one or more of said partitions; and
d) determining the intensity of a first detectable signal in said plurality of partitions
   (i) at a first determination timepoint during the exponential phase of the amplification, and
   (ii) at a second determination timepoint during a stationary phase of the amplification,

   wherein the first detectable signal correlates to the amount of said first target amplicons; and
   determining the number of partitions which are positive for said second target amplicons, wherein a positive partition has an intensity of a second detectable signal at a determination timepoint during the plateau phase of the amplification which is above a threshold value, wherein the second detectable signal correlates to the amount of said second target amplicons; and
e) calculating an initial number of first target nucleic acid molecules which contain said first target nucleic acid sequence that are present in said sample before said sample has been separated into said plurality of partitions, wherein said calculation is based on the relation between the intensity of the first detectable signal determined at the first determination timepoint and the intensity of the first detectable signal determined at the second determination timepoint; and
   calculating an initial number of second target nucleic acid molecules which contain said second target nucleic acid sequence that are present in said sample before said sample has been separated into said plurality of partitions based on the number or ratio of positive partitions,
   wherein each of said partitions in step b) contains on average less than five second target nucleic acid molecules; especially on average about one or fewer second target nucleic acid molecules.

Embodiment 113. A method of quantitating first target nucleic acid molecules and second target nucleic acid molecules in a sample comprising:
a) providing a sample comprising first target nucleic acid molecules which contain a first target nucleic acid sequence, and second target nucleic acid molecules which contain a second target nucleic acid sequence which is different from the first target nucleic acid sequence; and
b) separating said sample into a plurality of partitions; and
c) subjecting said plurality of partitions to multiple cycles of polymerase chain reaction (PCR cycles) for amplification of said first target nucleic acid sequence to produce first target amplicons in one or more of said partitions, and for amplification of said second target nucleic acid sequence to produce second target amplicons in one or more of said partitions; and
d) determining the intensity of a first detectable signal in said plurality of partitions
   (i) at a first determination timepoint during the exponential phase of the amplification, and
   (ii) at a second determination timepoint during a stationary phase of the amplification,

   wherein the first detectable signal correlates to the amount of said first target amplicons; and
   determining the number of partitions which are positive for said second target amplicons, wherein a positive partition has an intensity of a second detectable signal at a determination timepoint during the plateau phase of the amplification which is above a threshold value, wherein the second detectable signal correlates to the amount of said second target amplicons; and
e) calculating an initial number of first target nucleic acid molecules which contain said first target nucleic acid sequence that are present in said sample before said sample has been separated into said plurality of partitions, wherein said calculation is based on the relation between the intensity of the first detectable signal determined at the first determination timepoint and the intensity of the first detectable signal determined at the second determination timepoint; and
   calculating an initial number of second target nucleic acid molecules which contain said second target nucleic acid sequence that are present in said sample before said sample has been separated into said plurality of partitions based on the number or ratio of positive partitions,
   wherein each of said partitions in step b) contains on average less than five second target nucleic acid molecules; especially on average about one or fewer second target nucleic acid molecules,
   wherein the initial number of second target nucleic acid molecules which contain the second target nucleic acid sequence is calculated by application of Poisson statistics.

Embodiment 114. A method of quantitating first target nucleic acid molecules and second target nucleic acid molecules in a sample comprising:
a) providing a sample comprising first target nucleic acid molecules which contain a first target nucleic acid sequence, and second target nucleic acid molecules which contain a second target nucleic acid sequence which is different from the first target nucleic acid sequence; and
b) separating said sample into a plurality of partitions; and
c) subjecting said plurality of partitions to multiple cycles of polymerase chain reaction (PCR cycles) for amplification of said first target nucleic acid sequence to produce first target amplicons in one or more of said partitions, and for amplification of said second target nucleic acid sequence to produce second target amplicons in one or more of said partitions; and
d) determining the intensity of a first detectable signal in said plurality of partitions
   (i) at a first determination timepoint during the exponential phase of the amplification, and
   (ii) at a second determination timepoint during a stationary phase of the amplification,

   wherein the first detectable signal correlates to the amount of said first target amplicons; and
   determining the number of partitions which are positive for said second target amplicons, wherein a positive partition has an intensity of a second detectable signal at a determination timepoint during the plateau phase of the amplification which is above a threshold value, wherein the second detectable signal correlates to the amount of said second target amplicons; and
e) calculating an initial number of first target nucleic acid molecules which contain said first target nucleic acid sequence that are present in said sample before said sample has been separated into said plurality of partitions, wherein said calculation is based on the relation between the intensity of the first detectable signal determined at the first determination timepoint and the intensity of the first detectable signal determined at the second determination timepoint; and
   calculating an initial number of second target nucleic acid molecules which contain said second target nucleic acid sequence that are present in said sample before said sample has been separated into said plurality of partitions based on the number or ratio of positive partitions,
   wherein the initial number of second target nucleic acid molecules which contain the second target nucleic acid sequence is calculated by application of Poisson statistics.

Embodiment 115. A method of quantitating first target nucleic acid molecules and second target nucleic acid molecules in a sample comprising:
a) providing a sample comprising first target nucleic acid molecules which contain a first target nucleic acid sequence, and second target nucleic acid molecules which contain a second target nucleic acid sequence which is different from the first target nucleic acid sequence; and
b) separating said sample into a plurality of partitions; and
c) subjecting said plurality of partitions to multiple cycles of polymerase chain reaction (PCR cycles) for amplification of said first target nucleic acid sequence to produce first target amplicons in one or more of said partitions, and for amplification of said second target nucleic acid sequence to produce second target amplicons in one or more of said partitions; and
d) determining the intensity of a first detectable signal in said plurality of partitions
   (i) at a first determination timepoint during the exponential phase of the amplification, and
   (ii) at a second determination timepoint during a stationary phase of the amplification,

   wherein the first detectable signal correlates to the amount of said first target amplicons; and
   determining the number of partitions which are positive for said second target amplicons, wherein a positive partition has an intensity of a second detectable signal at a determination timepoint during the plateau phase of the amplification which is above a threshold value, wherein the second detectable signal correlates to the amount of said second target amplicons; and
e) calculating an initial number of first target nucleic acid molecules which contain said first target nucleic acid sequence that are present in said sample before said sample has been separated into said plurality of partitions, wherein said calculation is based on the relation between the intensity of the first detectable signal determined at the first determination timepoint and the intensity of the first detectable signal determined at the second determination timepoint; and
   calculating an initial number of second target nucleic acid molecules which contain said second target nucleic acid sequence that are present in said sample before said sample has been separated into said plurality of partitions based on the number or ratio of positive partitions,
   wherein each of said partitions in step b) contains on average less than five second target nucleic acid molecules; especially on average about one or fewer second target nucleic acid molecules,
   wherein the initial number of second target nucleic acid molecules which contain the second target nucleic acid sequence is calculated by application of Poisson statistics,
   wherein the second determination timepoint is during the plateau phase of the amplification reaction and the intensity of the second detectable signal is determined at the second determination timepoint.

Embodiment 116. A method of quantitating first target nucleic acid molecules and second target nucleic acid molecules in a sample comprising:
a) providing a sample comprising first target nucleic acid molecules which contain a first target nucleic acid sequence, and second target nucleic acid molecules which contain a second target nucleic acid sequence which is different from the first target nucleic acid sequence; and
b) separating said sample into a plurality of partitions; and
c) subjecting said plurality of partitions to multiple cycles of polymerase chain reaction (PCR cycles) for amplification of said first target nucleic acid sequence to produce first target amplicons in one or more of said partitions, and for amplification of said second target nucleic acid sequence to produce second target amplicons in one or more of said partitions; and
d) determining the intensity of a first detectable signal in said plurality of partitions
   (i) at a first determination timepoint during the exponential phase of the amplification, and
   (ii) at a second determination timepoint during a stationary phase of the amplification,

   wherein the first detectable signal correlates to the amount of said first target amplicons; and
   determining the number of partitions which are positive for said second target amplicons, wherein a positive partition has an intensity of a second detectable signal at a determination timepoint during the plateau phase of the amplification which is above a threshold value, wherein the second detectable signal correlates to the amount of said second target amplicons; and
e) calculating an initial number of first target nucleic acid molecules which contain said first target nucleic acid sequence that are present in said sample before said sample has been separated into said plurality of partitions, wherein said calculation is based on the relation between the intensity of the first detectable signal determined at the first determination timepoint and the intensity of the first detectable signal determined at the second determination timepoint; and
   calculating an initial number of second target nucleic acid molecules which contain said second target nucleic acid sequence that are present in said sample before said sample has been separated into said plurality of partitions based on the number or ratio of positive partitions,
   wherein each of said partitions in step b) contains on average less than five second target nucleic acid molecules; especially on average about one or fewer second target nucleic acid molecules,
   wherein the initial number of second target nucleic acid molecules which contain the second target nucleic acid sequence is calculated by application of Poisson statistics,
   wherein the second determination timepoint is during the plateau phase of the amplification reaction and the intensity of the second detectable signal is determined at the second determination timepoint,
   wherein the method is simultaneously performed for two or more different target nucleic acid sequences; wherein for each different target nucleic acid sequence respective target amplicons are produced in step c), and for each different target amplicon a different detectable signal is used in step d) which correlates to the amount the respective target amplicon.

Embodiment 117. The method according to any one of embodiments 111 to 116, wherein each of said partitions in step b) contains on average less than five second target nucleic acid molecules.

Embodiment 118. The method according to any one of embodiments 111 to 117, wherein each of said partitions in step b) contains on average about one or fewer second target nucleic acid molecules.

Embodiment 119. The method according to any one of embodiments 111 to 118, wherein the initial number of second target nucleic acid molecules which contain the second target nucleic acid sequence is calculated by application of Poisson statistics.

Embodiment 120. The method according to any one of embodiments 111 to 119, wherein the initial number of second target nucleic acid molecules is lower than the initial number of first target nucleic acid molecules.

Embodiment 121. The method according to any one of embodiments 111 to 120, wherein the threshold value in step d) is indicative of one second target nucleic acid molecule being present in the partition in step b).

Embodiment 122. The method according to any one of embodiments 111 to 121, wherein the second detectable signal is different from the first detectable signal.

Embodiment 123. The method according to any one of embodiments 111 to 122, wherein the second determination timepoint is during the plateau phase of the amplification reaction and the intensity of the second detectable signal is determined at the second determination timepoint.

Embodiment 124. The method according to any one of embodiments 1 to 123, wherein the sample
(i) is a biological sample; and/or
(ii) contains living, dead and/or lysed cells; and/or
(iii) is obtained from a biological organism; and/or
(iv) is a human sample; and/or
(v) is a wastewater sample; and/or
(vi) is a cell culture sample.

Embodiment 125. The method according to any one of embodiments 1 to 124, wherein the target nucleic acid molecules
(i) are DNA or RNA; and/or
(ii) encode a protein of interest; and/or
(iii) originate from a pathogen.

Embodiment 126. The method according to any one of embodiments 1 to 125, wherein the target nucleic acid sequence is indicative for the presence of a certain pathogen, organism, microorganism, vector, or plasmid

Embodiment 127. The method according to any one of embodiments 1 to 126, wherein the target nucleic acid sequence has a length of about 10 to about 2000 nucleotides.

Embodiment 128. The method according to any one of embodiments 1 to 127, wherein the partitions are liquid partitions or solid partitions.

Embodiment 129. The method according to any one of embodiments 1 to 128, wherein the partitions are droplets, liquid volumes in chambers, beads or surface areas on a chip.

Embodiment 130. The method according to any one of embodiments 1 to 129, wherein in step b) the sample is separated into at least 100 partitions, at least 1000 partitions, at least 10⁴, 10⁵, 10⁶, 10⁷ partitions.

Embodiment 131. The method according to any one of embodiments 1 to 130, comprising the further step of adding amplification reagents and/or detection reagents between steps a) and b) or between steps b) and c).

Embodiment 132. The method according to embodiment 131, wherein the amplification reagents comprise primer nucleic acid molecules specific for the target nucleic acid sequence, nucleotides and a polymerase.

Embodiment 133. The method according to embodiment 131 or 132, wherein the detection reagents comprise a label, especially an optical label, which emits the detectable signal in the presence of the target amplicon.

Embodiment 134. The method according to any one of embodiments 1 to 133, wherein each of the PCR cycles comprises the steps of denaturing nucleic acid duplexes, hybridizing primer nucleic acid molecules with the target nucleic acid sequence, and elongating the hybridized primer nucleic acid molecules by a polymerase.

Embodiment 135. The method according to any one of embodiments 1 to 134, wherein in step c) the plurality of partitions are subjected to at least 20 PCR cycles, preferably at least 25, at least 30, at least 35, at least 40, at least 45, at least 50, at least 55, at least 60 PCR cycles; at least 65 PCR cycles, at least 70 PCR cycles, at least 75 PCR cycles, at least 80 PCR cycles.

Embodiment 136. The method according to any one of embodiments 1 to 135, wherein the method is simultaneously performed for two or more different target nucleic acid sequences.

Embodiment 137. The method according to embodiment 136, wherein for each different target nucleic acid sequence respective target amplicons are produced in step c), and for each different target amplicon a different detectable signal is used in step d) which correlates to the amount the respective target amplicon.

Embodiment 138. A system for performing the method according to any one of embodiments 1 to 137, comprising:
(a) at least one processor for performing the method according to any one of embodiments 1 to 137; and
(b) a memory encoded with instructions to perform the method according to any one of embodiments 1 to 137.

Embodiment 139. The system according to embodiment 138, further comprising
(c) a display;
(d) a thermal cycler;
(e) an optical system;
(f) a storage device; and
(g) optionally an input device.

Embodiment 140. The system according to embodiment 138 or 139, wherein the components (a) to (b) or (a) to (g) are connected to communicate and process information.

Embodiment 141. A computer-readable storage medium encoded with instructions, executable by a processor, for performing the method according to any one of embodiments 1 to 137.

### EXAMPLES

It should be understood that the following examples are for illustrative purpose only and are not to be construed as limiting this invention in any manner.

### Abbreviations:

- dPCR: digital polymerase chain reaction
- NTC: no template control
- PCR: polymerase chain reaction
- RFU: Real Fluorescence Unit
- Ct: Cycle threshold

The following examples were performed with the QIAcuity system (QIAGEN, Hilden) and corresponding kits. With respect to sample preparation, consumables, buffers and reagents, thermocycling, imaging, and further devices required reference is made to the QIAcuity User Manual (QIAGEN, Hilden, November 2023). The QIAcuity Software Suite (QIAGEN, Hilden) was used to assess mean RFU per concentration per cycle.

### Example 1: dPCR with intermediary imaging steps

In dPCR, each well is divided into lots of sub-samples, i.e. partitions. For each of those partitions an end point PCR is executed and a signal is measured in a certain physical position. Each partition is represented according to its respective spatial location and its signal intensity. The signal intensity (RFU) depends on the amplification reaction meaning that partitions where an amplification reaction occurred are represented by a bright signal while for partitions where no amplification reaction occurred no signal is detected. The intensity of the signal depends on the number of target nucleic acid molecules present in the partition. According to the specific characteristics of the signal, the partition is classified as positive (containing a target ucleic acid molecule) or negative (not containing a target nucleic acid molecule).

dPCR was performed in a 26K 24-well QIAcuity Nanoplate using the QIAcuity probe mastermix. A dilutions series ranging from 320,000 copies/µl to 800 copies/µl was prepared from synthetic DNA template (BRAF V600 mutation detection assay gBlocks). In contrast to conventional dPCR, multiple intermediate imaging steps were performed. The QIAcuity Nanoplate was imaged after 5, 10, 15, 20, 25, 30, 40, 50, and 60 PCR cycles (table 1).

**Table 1: Mean RFU value measurement**

| Plate | Concentration (copies/µL) | Mean RFU at cycle # | | | | | | | | | |
|---|---|---|---|---|---|---|---|---|---|---|---|
| | | 0 | 5 | 10 | 15 | 20 | 25 | 30 | 40 | 50 | 60 |
| row A | 320000 | 25,48 | 28,5 | 29,5 | 33,54 | 75,46 | 126,39 | 153,46 | 169,48 | 173,39 | 172,38 |
| row B | 160000 | 26,49 | 28,48 | 29,48 | 32,02 | 63,44 | 113,53 | 144,51 | 167,49 | 173,41 | 173,41 |
| row C | 80000 | 26,49 | 28,53 | 28,52 | 29,51 | 51,52 | 102,44 | 136,43 | 165,48 | 172,52 | 173,47 |
| row D | 40000 | 26,45 | 27,51 | 28,5 | 28, 51 | 43, 51 | 91,42 | 131,46 | 164,53 | 170,45 | 173,44 |
| row E | 20000 | 27,49 | 27,48 | 27,52 | 28,52 | 36,47 | 81,49 | 123,47 | 162,94 | 172,43 | 173,48 |
| row F | 10000 | 27,51 | 27,51 | 28,52 | 27,51 | 32,48 | 71,51 | 118,4 | 162,47 | 173,53 | 174,52 |
| row G | 800 | 27,51 | 27,51 | 27, 5 | 28 | 28, 51 | 45,5 | 97,4 | 156,44 | 171,47 | 173,39 |
| row H | 0 | 27,49 | 27,49 | 27,48 | 27,46 | 27,55 | 27,56 | 27,55 | 28,53 | 27,47 | 27,47 |

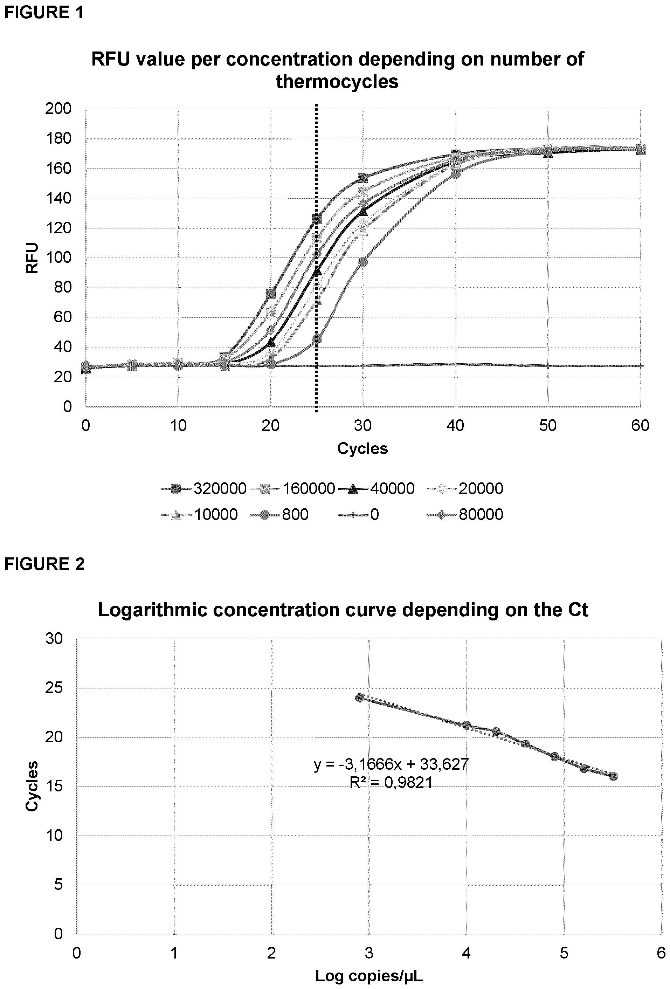

The mean RFU per cycle for each concentration is displayed in figure 1.

An artificial threshold was set at 40 RFU and a Ct was defined for each template concentration, wherein the Ct corresponds to the cycle at which the RFU is cutting the artificial threshold and starts exponential fluorescence increase. Based on those values, Ct and known concentration, a standard curve was created (figure 2).

The results show that a clear discrimination can be done between the different concentrations of the dilution series. The discrimination between the different template concentrations was clearest during exponential phase (roughly at cycle 25, see dotted line in figure 1) while the different concentrations could no longer be discriminated after reaching the plateau phase (roughly at cycle 50) due to saturation (figure 1).

For each concentration the RFU ratio was calculated between cycle 25 and cycle 50 by dividing the mean RFU from cycle 25 by the mean RFU from cycle 50.

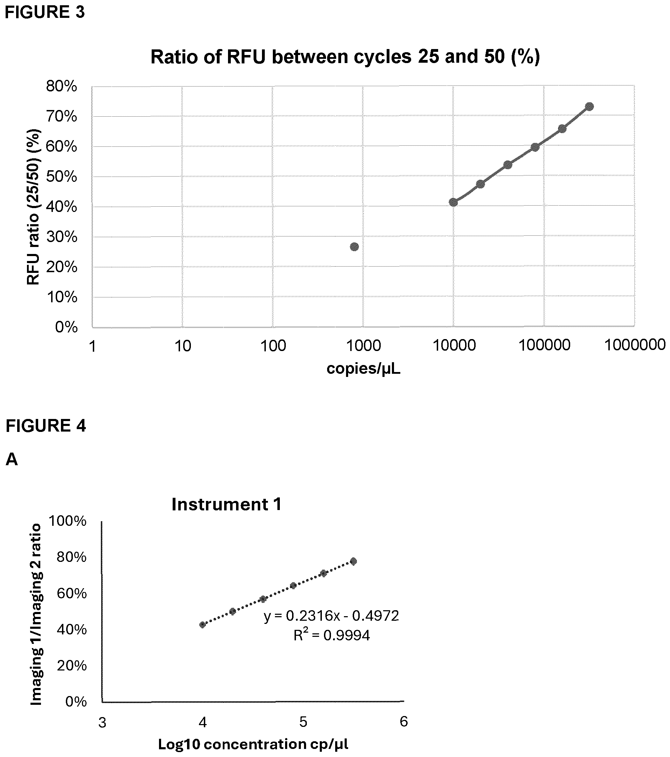

The resulting distribution showed that dPCR RFU values are linear and depend on the initial template concentration (figure 3). Accordingly, an additional intermediate imaging step during exponential phase in the dPCR workflow, for example at cycle 25, allowed to determine the concentration of the template by linking the observed RFU ratio to a corresponding RFU ratio of a known template concentration. To that end at least one calibration run can be done to assess the dependency of the template concentration on the ratio of the RFU at an intermediate cycle to the RFU at the end point. This method allowed to calculate the concentration of high template samples even if saturation is reached in end point measurement.

### Example 2: Quantification of high copy number target nucleic acid

The QIAcuity performs a fully automated processing of the QIAcuity Nanoplates, including all necessary steps of plate priming, sealing of partitions, thermocycling, and image analysis. Depending on the plate type, up to 8, 24, or 96 samples per plate can be analyzed. For high sensitivity applications, the QIAcuity Nanoplate 26K 8- or 24-well is used. The number of in parallel processable plates depends on the instrument configuration. The QIAcuity controls all integrated modules, including a robotic gripper for plate handling, a partitioning module, a PCR thermocycler, and a fluorescence imaging module.

The method using the intermediate imaging step was tested with a 24 well 26k Nanoplate using QIAcuity Advanced Probe MasterMix without RT. A synthetic DNA template (BRAF V600 mutation detection assay gBlocks) serial dilution in a concentration of 320,000, 160,000, 80,000, 40,000, 20,000, 10,000 and 1,000 copies/µl was used as standard to create a standard curve according to example 1. The standard curve was used to calculate the concentration of target nucleic acid molecules in the test samples. Four test samples were added to the test plate layout at a target concentration of 80,000, 100,000, 15,000 and 9,000 copies/µl.

The target of interest was amplified with specific primers (0.4µM final concentration) and detected using a specific TaqMan probe label with FAM fluorophore (0.8µM final concentration). For this experiment, each concentration was tested in duplicates and NTC (no template control) wells were pipetted. A first PCR denaturation step (2min at 95°C) was performed in thermocycler and followed by 25 PCR cycles (15s 95°C and 30s 60°C). Subsequently, a first imaging step was performed. The nanoplates were then placed back in the thermocycler and another 25 PCR cycles (15s 95°C and 30s 60°C) were performed, followed by a last imaging step on QIAcuity system. The target of interest was detected using GREEN filter pair, with an exposure time of 500 ms and gain of 6. The same experiment was performed, side by side, on 3 different QIAcuity instruments. 3 plates were run per instruments.

**Table 2: dPCR conditions**

| | | |
|---|---|---|
| 2 min | | 95 °C |
| 25x | | |
| | 15 sec | 95 °C |
| | 30 sec | 60 °C |
| Imaging | | |
| 25 x | | |
| | 15 sec | 95 °C |
| | 30 sec | 60 °C |
| Imaging | | |

Mean RFU ratio 25/50 was calculated for each standard as well as for the test samples, by dividing the mean RFU from the first imaging step (after 25 PCR cycles, "imaging 1") by the mean RFU from the second imaging step (after 50 PCR cycles, "imaging 2").

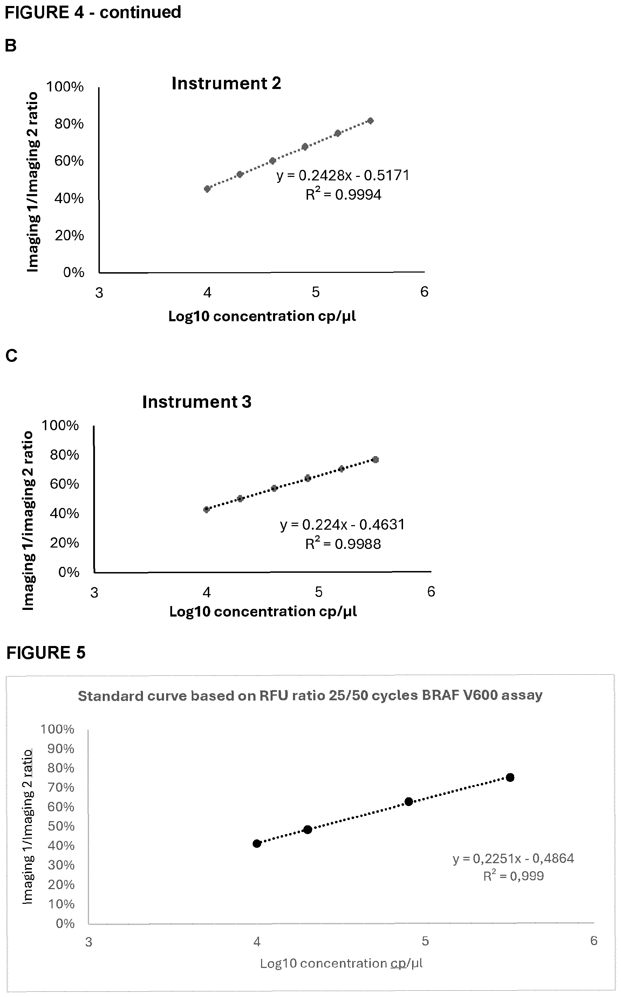

A standard curve was created based on the ratio and concentration (log cop/µL) of each standard concentration. The standard curve of each instrument is displayed in figure 4. For the three instruments the r² values were above 0.998.

The linear curve formular was then used to back calculate the concentration of the test samples and standard derivation was calculated for each template concentration and instrument (see table 3).

**Table 3: Back calculation of test sample concentration**

| Instrument | Concentration (copy/µl) | Sample | Measured concentration (copy/µl) | SD measured concentration |
|---|---|---|---|---|
| 1 | 80000 | 1 | 84568.2 | 3884.9 |
| | 100000 | 2 | 99289.1 | 4696.0 |
| | 15000 | 3 | 15435.0 | 311.4 |
| | 9000 | 4 | 9228.9 | 249.5 |
| 2 | 80000 | 1 | 82806.3 | 1792.0 |
| | 100000 | 2 | 91754.5 | 4474.6 |
| | 15000 | 3 | 14879.7 | 335.5 |
| | 9000 | 4 | 9339.7 | 336.1 |
| 3 | 80000 | 1 | 82524.3 | 1918.4 |
| | 100000 | 2 | 98892.2 | 6203.1 |
| | 15000 | 3 | 15218.1 | 229.6 |
| | 9000 | 4 | 9458.6 | 316.4 |

The results demonstrated that the concentration of a target nucleic acid in a sample can accurately be determined using dPCR with an additional intermediate imaging step during the exponential phase of the amplification and calculating the ratio of the mean RFU of said intermediate imaging and the mean RFU of the end point imaging.

### Example 3: Simultaneous quantification of high and low copy number target nucleic acids

This method was tested with a 24 well 26k Nanoplate using QIAcuity Advanced Probe MasterMix without RT. A serial dilution of a synthetic DNA template (BRAF V600 mutation detection assay gBlocks) in concentrations of 320,000, 160,000, 80,000, 40,000, 20,000, 10,000 and 1,000 copies/µl was added. Mixes were prepared in duplex with EGFR assay and respective EGFR gDNA template at 20 copies/µl. BRAF V600 serial dilution wells were used as standard to create a standard curve and calculate the concentration of the test samples. Targets of interest were amplified with specific primers (0.4µM final concentration) and detected using a specific TaqMan probe label with FAM fluorophore for the BRAF V600 target and with HEX fluorophore for the EGFR target (both at 0.8µM final concentration). For this experiment, each concentration was tested in triplicates and NTC wells (Non Template Control) were pipetted. A usual first PCR denaturation step of 2 min at 95°C was performed in a thermocycler and followed by 25 PCR cycles (15s 95°C and 30s 60°C), then a first imaging step was performed. The nanoplates were then placed back in thermocycler to perform addition 25 PCR cycles (15s 95°C and 30s 60°C) again followed by an end point imaging step on QIAcuity system. Targets of interest were detected using a GREEN and YELLOW filter pair with exposure time of 500 ms and gain of 6.

Mean RFU ratio (25/50) was calculated for each standard as well as for the test samples by dividing the mean RFU from the first imaging step (after 25 PCR cycles, "imaging 1") by the mean RFU from the second imaging step (after 50 PCR cycles, "imaging 2").

BRAF V600 assay standard curve was created based on the RFU ratio and concentration (log cop/µL) of each standard concentration (Figure 5).

EGFR concentration was measured with the second imaging step using standard dPCR measurement with QIAcuity Software Suite absolute quantification. Table 4 shows the concentration of the EGFR template being 17.78 cop/µl.

**Table 4: Calculation of the low copy test sample concentration using Poisson statistics**

| Row | dPCR mean concentration (copy/µl) | SD |
|---|---|---|
| A | 17.61 | 0.33 |
| B | 18.22 | 0.52 |
| C | 17.30 | 0.40 |
| D | 17.41 | 0.71 |
| E | 17.86 | 0.96 |
| F | 17.93 | 0.77 |
| G | 17.49 | 1.27 |
| H | 18.41 | 0.11 |
| mean | 17.78 | 0.81 |

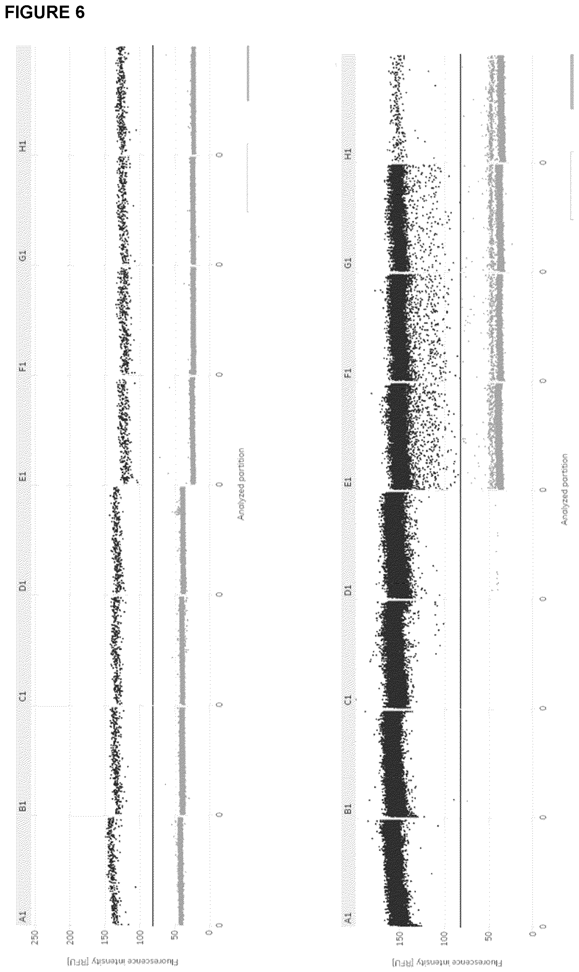

Figure 6 shows the signal detected by QIAcuity software Suite in the second imaging step (after 50 cycles) for both target, EGFR and BRAF V600.

### Example 4: Quantification of target nucleic acids over a broad concentration range

This method was tested with a 24 well 26k Nanoplate using QIAcuity Advanced Probe MasterMix without RT. A serial dilution of a synthetic DNA template (BRAF V600 mutation detection assay gBlocks) in concentrations of 320,000, 160,000, 80,000, 40,000, 20,000, 10,000 and 1,000 copies/µl was added. Serial dilution wells were used as standard to create a standard curve and estimate the concentration of the test samples. Target of interest was amplified with specific primers (0.4µM final concentration) and detected using a specific TaqMan probe label with FAM fluorophore (0.8µM final concentration). For this experiment, each concentration was tested in duplicates and NTC wells (with no DNA template) were pipetted. A usual first PCR denaturation step of 2 min at 95°C was performed in a thermocycler and followed by 25 PCR cycles (15s 95°C and 30s 60°C), then a first imaging step was performed. The nanoplates were then placed back in thermocycler to perform addition 25 PCR cycles (15s 95°C and 30s 60°C) again followed by an end point imaging step on QIAcuity system. The target of interest was detected using a GREEN filter with exposure time of 500 ms and gain of 6. The experiment was performed, with QIAcuity One-plate instrument.

Mean RFU ratio 25/50 was calculated for each standard as well as for the test samples by dividing the mean RFU from the first imaging step (after 25 PCR cycles) by the mean RFU from the second imaging step (after 50 PCR cycles).

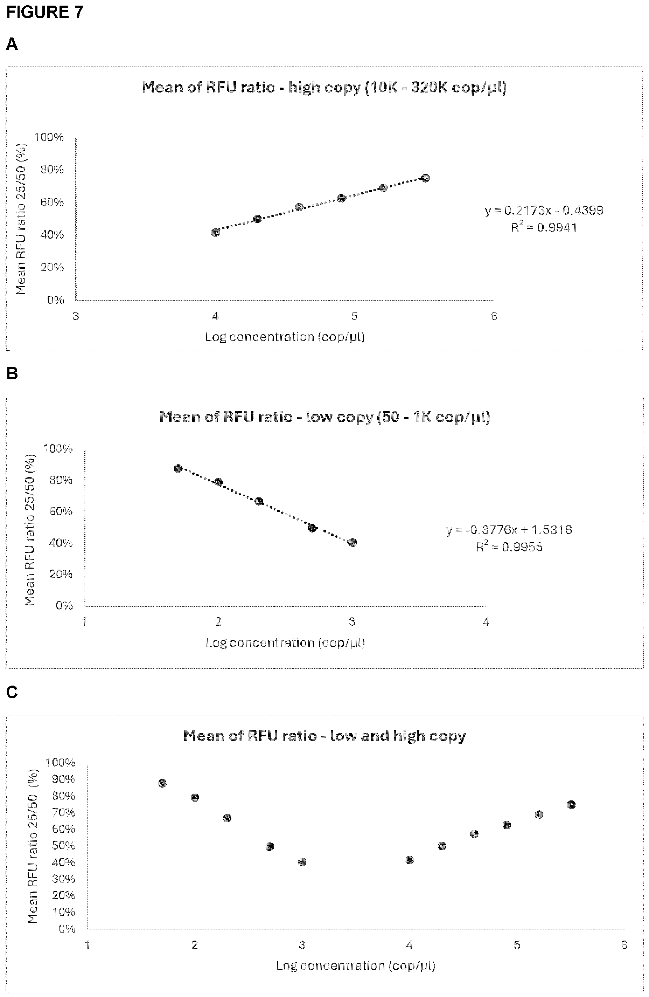

Mean RFU ratio 25/50 (%) was plotted against log concentration (copies/µl). The graphs in figure 7 show two different linearity trends, one for a lower concentration range (from 50 to 1,000 copies/µl) and one for a higher concentration range (from 10,000 to 320,000 copies/µl). The RFU ratio in the low concentration range decreases with increasing target concentration because here the first imaging step is still in the baseline phase and the second imaging step is in the exponential phase.

Two standard curves were created based on RFU ratio and concentration (log cop/µL) for both concentration ranges. Standard curves are displayed in Figure 7 A and B. For both standard curves the r² value is above 0.994.

## Claims

1. A method of quantitating target nucleic acid molecules in a sample comprising:
a) providing a sample comprising target nucleic acid molecules which contain a target nucleic acid sequence; and
b) separating said sample into a plurality of partitions; and
c) subjecting said plurality of partitions to multiple cycles of polymerase chain reaction (PCR cycles) for amplification of said target nucleic acid sequence to produce target amplicons in one or more of said partitions; and
d) determining the intensity of a detectable signal in said plurality of partitions
(i) at a first determination timepoint during the exponential phase of the amplification, and
(ii) at a second determination timepoint during a stationary phase of the amplification,
wherein the detectable signal correlates to the amount of said target amplicons; and
e) calculating an initial number of target nucleic acid molecules which contain said target nucleic acid sequence that are present in said sample before said sample has been separated into said plurality of partitions, wherein said calculation is based on the relation between the intensity of the detectable signal determined at the first determination timepoint and the intensity of the detectable signal determined at the second determination timepoint.

2. The method according to claim 1, wherein each of said partitions in step b) contains on average more than one target nucleic acid molecules, especially on average five or more target nucleic acid molecules.

3. The method according to claim 1 or 2, wherein the portion of said plurality of partitions that contains zero target nucleic acid molecules is 0.2% or less, preferably 0.02% or less.

4. The method according to any one of claims 1 to 3, wherein the first determination timepoint is during or directly after any one of PCR cycles 10 to 35; preferably any one of PCR cycles 20 to 30, for example PCR cycle 25.

5. The method according to any one of claims 1 to 4, wherein the second determination timepoint is
(i) during the plateau phase of the amplification; and/or
(ii) during or directly after any one of PCR cycles 30 to 80; preferably any one of PCR cycles 45 to 55, for example PCR cycle 50.

6. The method according to any one of claims 1 to 4, wherein the second determination timepoint is
(i) during the baseline phase of the amplification; and/or
(ii) during or directly after any one of PCR cycles 1 to 20; preferably any one of PCR cycles 1 to 15, for example PCR cycle 1 or PCR cycle 5.

7. The method according to any one of claims 1 to 6, wherein the first determination timepoint and the second determination timepoint are separated by at least 3 PCR cycles.

8. The method according to any one of claims 1 to 7, wherein in step d) a common intensity of the detectable signal is determined for all partitions of the plurality of partitions, or wherein individual intensities of the detectable signal are measured for each partition of the plurality of partitions, wherein the individual intensities of the partitions are added up to provide a total intensity, or wherein the individual intensities of the partitions are used to calculate a mean or median intensity.

9. The method according to any one of claims 1 to 8, wherein the intensity of the detectable signal is only determined at the first determination timepoint and at the second determination timepoint, and optionally at a third determination timepoint during the baseline phase of the amplification.

10. The method according to any one of claims 1 to 9, wherein the calculation involves
(i) calculating a relative value by dividing the intensity of the detectable signal determined at the first determination timepoint by the intensity of the detectable signal determined at the second determination timepoint; and
(ii) comparing the relative value to reference relative values and/or a reference curve;
wherein optionally the reference relative values and/or the reference curve are based on at least two reference samples comprising a known number of reference nucleic acid molecules, wherein reference amplicons are produced from the reference nucleic acid molecules by the same method steps b) and c) as for the target amplicons, and wherein reference relative values are determined by the same method steps d) and e) as the relative value for the target amplicons; and
wherein optionally the reference nucleic acid molecules contain the same target nucleic acid sequence as the target nucleic acid molecules, and wherein the reference amplicons are produced by amplification of said target nucleic acid sequence present on the reference nucleic acid molecules; and
wherein optionally the reference curve is a plot of the reference relative values of the at least two reference samples against the known number of reference nucleic acid molecules in the at least two reference samples.

11. A method of quantitating first target nucleic acid molecules and second target nucleic acid molecules in a sample comprising:
a) providing a sample comprising first target nucleic acid molecules which contain a first target nucleic acid sequence, and second target nucleic acid molecules which contain a second target nucleic acid sequence which is different from the first target nucleic acid sequence; and
b) separating said sample into a plurality of partitions; and
c) subjecting said plurality of partitions to multiple cycles of polymerase chain reaction (PCR cycles) for amplification of said first target nucleic acid sequence to produce first target amplicons in one or more of said partitions, and for amplification of said second target nucleic acid sequence to produce second target amplicons in one or more of said partitions; and
d) determining the intensity of a first detectable signal in said plurality of partitions
(i) at a first determination timepoint during the exponential phase of the amplification, and
(ii) at a second determination timepoint during a stationary phase of the amplification,
wherein the first detectable signal correlates to the amount of said first target amplicons; and
determining the number of partitions which are positive for said second target amplicons, wherein a positive partition has an intensity of a second detectable signal at a determination timepoint during the plateau phase of the amplification which is above a threshold value, wherein the second detectable signal correlates to the amount of said second target amplicons; and
e) calculating an initial number of first target nucleic acid molecules which contain said first target nucleic acid sequence that are present in said sample before said sample has been separated into said plurality of partitions, wherein said calculation is based on the relation between the intensity of the first detectable signal determined at the first determination timepoint and the intensity of the first detectable signal determined at the second determination timepoint; and
calculating an initial number of second target nucleic acid molecules which contain said second target nucleic acid sequence that are present in said sample before said sample has been separated into said plurality of partitions based on the number or ratio of positive partitions.

12. The method according to claim 11, wherein each of said partitions in step b) contains on average less than five second target nucleic acid molecules; especially on average about one or fewer second target nucleic acid molecules.

13. The method according to claim 11 or 12, wherein the initial number of second target nucleic acid molecules which contain the second target nucleic acid sequence is calculated by application of Poisson statistics.

14. The method according to any one of claims 11 to 13, wherein the second determination timepoint is during the plateau phase of the amplification reaction and the intensity of the second detectable signal is determined at the second determination timepoint.

15. The method according to any one of claims 1 to 14, wherein the method is simultaneously performed for two or more different target nucleic acid sequences; wherein for each different target nucleic acid sequence respective target amplicons are produced in step c), and for each different target amplicon a different detectable signal is used in step d) which correlates to the amount the respective target amplicon.

16. A system for performing the method according to any one of claims 1 to 15, comprising:
(a) at least one processor for performing the method according to any one of claims 1 to 15;
(b) a memory encoded with instructions to perform the method according to any one of claims 1 to 15;
(c) optionally a display;
(d) optionally a thermal cycler;
(e) optionally an optical system;
(f) optionally a storage device; and
(g) optionally an input device.

17. A computer-readable storage medium encoded with instructions, executable by a processor, for performing the method according to any one of claims 1 to 15.
